# EUROPEAN PATENT SPECIFICATION

(11) **EP 4 469 560 B1**
(45) Date of publication and mention of the grant of the patent: **11.03.2026**
(21) Application number: 23701495.6
(22) Date of filing: 23.01.2023
(51) Int. Cl.: C12N 5/07, C12N 5/071, C12N 7/00

(54) **AN IMPROVED CELL CULTURE MEDIUM FOR CRUSTACEAN CELLS**
VERBESSERTES ZELLKULTURMEDIUM FÜR KRUSTENTIERZELLEN
MILIEU DE CULTURE CELLULAIRE AMÉLIORÉ POUR CELLULES DE CRUSTACÉS

(30) Priority: 26.01.2022 EP 22153353
(43) Date of publication of application: 04.12.2024
(73) Proprietor: Universiteit Gent, 9000 Gent (BE)
(72) Inventor: NAUWYNCK, Hans, 9930 Zomergem (BE); RAKHSHANINEJAD, Mostafa, 9820 Merelbeke (BE)
(86) International application number: PCT/EP2023/051493
(87) International publication number: WO 2023/144061

(56) References cited:
- SIVAKUMAR S ET AL: "Medium optimization and characterization of cell culture system fromPenaeus vannameifor adaptation of white spot syndrome virus (WSSV)", JOURNAL OF VIROLOGICAL METHODS, vol. 270, 16 April 2019 (2019-04-16), pages 38 - 45, XP085708632, ISSN: 0166-0934, DOI: 10.1016/J.JVIROMET.2019.04.016
- JAYESH P. ET AL: "A novel medium for the development of in vitro cell culture system from Penaeus monodon", vol. 65, no. 3, 1 May 2013 (2013-05-01), Dordrecht, pages 307 - 322, XP055934794, ISSN: 0920-9069, Retrieved from the Internet <URL:https://www.ncbi.nlm.nih.gov/pmc/articles/PMC3597175/pdf/10616_2012_Article_9491.pdf> DOI: 10.1007/s10616-012-9491-9
- CHEN S. N. ET AL: "Establishment of cell culture systems from penaeid shrimp and their susceptibility to white spot disease and yellow head viruses", METHODS IN CELL SCIENCE 21: 199-206 (1999)., 30 October 1999 (1999-10-30), pages 199 - 206, XP055934789, Retrieved from the Internet <URL:https://link.springer.com/content/pdf/10.1023/A:1009885929335.pdf> [retrieved on 20220623]
- LI WENFENG ET AL: "Characterization of a primary cell culture from lymphoid organ of Litopenaeus vannamei and use for studies on WSSV replication", AQUACULTURE, vol. 433, 1 September 2014 (2014-09-01), Amsterdam, NL, pages 157 - 163, XP055934951, ISSN: 0044-8486, DOI: 10.1016/j.aquaculture.2014.05.046
- SHIMIZU CHISATO ET AL: "HEMOLYMPH ANALYSIS AND EVALUATION OF NEWLY FORMULATED MEDIA FOR CULTURE OF SHRIMP CELLS (PENAEUS STYLIROSTRIS)", IN VITRO CELLULAR & DEVELOPMENTAL BIOLOGY. ANIMAL, 1 June 2001 (2001-06-01), Germany, pages 322 - 329, XP055934755, Retrieved from the Internet <URL:https://link.springer.com/content/pdf/10.1007/BF02577565.pdf> [retrieved on 20220623], DOI: 10.1290/1071-2690(2001)037<0322:HAAEON>2.0.CO;2
- LUEDEMAN RENÉA. ET AL: "Development of an in vitro primary cell culture system from the penaeid shrimp, Penaeus stylirostris and Penaeus vannamei", AQUACULTURE, vol. 101, no. 3-4, 1 February 1992 (1992-02-01), Amsterdam, NL, pages 205 - 211, XP055934957, ISSN: 0044-8486, DOI: 10.1016/0044-8486(92)90024-F

## Description

### Technical field of invention

The present invention relates to a cell culture medium which sustains the growth of cells obtained from a crustacean in vitro. Indeed, the present invention discloses a cell culture medium comprising a specific salt composition, in part responsible for the correct osmolality and pH, a specific amino acid and vitamin composition, energy sources such as glucose, and crustacean hemolymph. The present invention further discloses that said cell culture medium is capable to keep cells obtained from shrimp and lobster viable for long periods of time even after passaging the cells into subcultures and induces said cells to proliferate. The cultured cells are for example useful to perform physiological studies on certain genes (by genetic engineering) and to do research and diagnosis on crustacean pathogens.

### Background art

Cell culture is one of the major research tools in life sciences (Milton, 2014). It is the general term used to remove cells, tissues or organs from an animal or plant and their subsequent placement into an artificial environment conducive to their survival and/or proliferation. Cell cultures are one of the significant tools used in cellular and molecular biology, providing excellent model systems for studying the normal physiology and biochemistry of cells (e.g. metabolic studies, aging), the effects of drugs and toxic compounds on cells, and mutagenesis and carcinogenesis (Weltin et al., 2014). It is also used in drug screening and development and large-scale manufacturing of biological compounds (e.g., vaccines, therapeutic proteins) (Haab, 2005). The major advantage of using cell cultures for any of these applications is the consistency and reproducibility of results obtained from a batch of clonal cells (Freshnay et al., 2000; Butler, 2004).

Normal cells divide only a limited number of times before losing their ability to proliferate, which is a genetically determined event known as senescence. However, some cell lines become immortal through a process called transformation, which can occur spontaneously or can be chemically or virally induced (Gudjonsson et al., 2004). When a cell culture undergoes transformation and acquires the ability to divide indefinitely, it becomes a continuous cell line (Todaro et al., 1965).

Until now, there is no continuous crustacean cell line available. Cell culture techniques for crustaceans have been developed to permit the isolation, growth and identification of viral pathogens. To date, emphasis has been placed on the commercially important penaeids (Chang et al., 1989; Toullec, 1999; Claydon, 2009).

The first cell culture system from tissues of penaeid shrimp was reported in 1986 (Chen et al., 1986). This cell culture was derived from the gonad and heart tissues of *P. monodon.* Subsequently, especially when the white spot syndrome virus was first detected in 1992 in Taiwan and later on in most Southeast Asian countries (Kasornchandra et al., 1995), some experiments were done on primary cell cultures from penaeid shrimps (Luedeman & Lightner, 1992; Dantas-Lima et al., 2012; Li et al., 2014; Ma et al., 2017). Different media and medium supplements (sera, tissue extracts, growth factors...) were applied in primary cell culture. However, getting the cells in mitosis and subculturing the primary shrimp cells have been found to be the most difficult tasks (Han et al., 2013). As a result, subcultured primary cell cultures (limited number of subcultures) and continuous cell lines (which can be subcultured without limitation) are not available. Although subculturing shrimp primary cells have been reported, the capability of shrimp cells to proliferate was never demonstrated (Han et al., 2013). In many cases, these cells were not shrimp cells but cells from contaminating micro-organisms (Hsu et al., 1995). In addition, only a few reports exist on the infection of cell cultures with the white spot syndrome virus (WSSV) (Tapay et al., 1995). In brief, despite several decades of research efforts, subculturing shrimp cells with the purpose to let them proliferate and to make continuous cell lines from crustaceans such as penaeid shrimp still has to be developed (Jose et al., 2010).

Most trials to make primary cultures of crustacean tissues are derived from penaeid shrimp. The most popular species of penaeid shrimp that was used for cell cultures in the early days was *Penaeus monodon* (Hsu et al., 1995). Since the culture of *P. vannamei* expanded rapidly over most parts of Asia after 1996 (Chamberlain, 2010), this species has now become the most important cultured penaeid species. Within this period, *P. vannamei* has been applied in cell culture studies since 1999 (Dantas-Lima et al., 2012; Han et al., 2013). *P. japonicus* (Itami et al., 1999) and *P. stylirostris* (Shike et al., 2000) were also used in shrimp cell culture. Other species including *P. chinensis, P. indicus, P. merguiensis, P. orientates, P. aetecus* and *P. ensis* have been barely used due to their limited living spaces and low production in aquaculture. So far, little research has been done on culturing cells from lobster (*Homarus americanus*), an economically important species (Stepanyan et al., 2004).

The tissue used for primary cell cultures is considered an important first selection step for successfully developing cell lines. In the studies of penaeid shrimp cell cultures, tissue sources are very variable. The lymphoid organ is the most commonly used cell source for making cell cultures (Tapay et al., 1995; Wang et al., 2000; Han et al., 2013). It is followed by the ovary (George & Dhar, 2010), hepatopancreas (Chen et al., 1986), hemocytes (Dantas-Lima et al., 2012) and heart (Chen and Wang, 1999). Cell cultures of shrimp derived from muscle, nerves (Wang et al., 2000), epidermis (Toullec, et al., 1996), hematopoietic tissue, and gills (Hsu et al., 1995) have also been reported in some studies. Other tissues, such as the gut, eyestalks (George & Dhar, 2010), and embryos, were occasionally used as tissue sources in cell cultures (Fan et al., 2002).

Basic environmental requirements for cells to grow optimally are a balanced salt composition that controls the osmolality and pH, the temperature, a substrate for cell attachment and an appropriate growth medium. The most important and crucial step in cell culture is selecting an appropriate growth medium for in vitro cultivation. A growth medium or culture medium is a liquid or gel designed to support the growth of microorganisms, cells or small plants (Helgason & Cindy, 2005). Cell culture media generally comprise an appropriate source of energy and compounds which regulate the cell cycle. A typical culture medium is composed of a combination of inorganic salts, amino acids, vitamins, energy sources and serum as a source of growth factors, hormones and attachment factors. The medium helps in maintaining the pH and osmolality (Lee et al., 2011).

Culture medium plays a crucial role in developing primary cell cultures and cell lines. A proper culture medium can significantly improve cell behavior. Different media have different characteristics with regard to the concentrations of salts, carbohydrates, amino acids and vitamins and buffer (McKee & Svetlana, 2017). In the field of *in vitro* cell cultures derived from penaeid shrimps, there is very limited knowledge of the detailed nutritional requirements. Thus, specifically modified media designed for mammals and insects have been generally applied for cell cultures of penaeid shrimp (Toullec, 1999). Leibovitz's 15 (L-15), M-199 and Grace's insect medium have been reported to fit cell cultures from penaeid shrimp (Luedeman & Lightner, 1992; George & Dhar, 2010). Besides these, other media such as DMEM, MEM, CMRL, MPS, F12, NCTC, Excell 401 and TC-100 have been tested in studies of shrimp cell cultures with the goal to improve either growth or survival of the cells, but the results were not satisfactory (George & Dhar, 2010). At present, no efforts were made to make a crustacean-specific medium.

Inorganic salts in the media help to maintain the osmotic balance and help to regulate the membrane potential by providing sodium, potassium, magnesium, and calcium ions (Yao & Yuta, 2017). Most of the penaeid shrimp live in marine environments, and cells are adapted to grow in higher osmolality compared to mammalian cells. To culture the crustacean cell, the osmolality of culture media varies greatly in between studies, ranging from 470 mmol/kg (Hsu et al., 1995) up to 1025 mmol/kg (Luedeman & Lightner, 1992; Nadala et al., 1993; Kasornchandra et al., 1999; Li et al., 2007). It is important to mention here that a small change in the osmolality of the environment in the range of ±50 mmol/kg imposes osmotic stress upon cells that affect the concentration and stability of macromolecules (Timasheff, 1993), the rate of biochemical reactions, and cell function as a whole (Somero & Yancey, 1997). Cells respond to osmotic stress with a variety of osmoregulatory responses, including cell volume regulation (Hoffmann & Dunham, 1995), restoration of intracellular inorganic ion homeostasis by compatible organic osmolytes (Burg et al., 1997), and compensation of macromolecular function (van Why & Siegel, 1998).

Important to mention here is that the concentration of inorganic salts and their composition that ultimately lead to a final osmolality value is much more critical than the value of osmolality because inorganic ions are crucial in different physiological pathways (Yao & Yuta, 2017). A correlation between osmolality and Cl⁻, Na⁺, K⁺, and Ca²⁺ concentrations have been observed in mud crab *Scylla serrata* in relation to size and molt cycle (Chen and Chia, 1997).

Under physiological conditions, the regulation of the cell volume is of importance to most cell types and therefore, cells possess a diverse repertoire of ion channels and pumps through which ion gradients, particularly K⁺, Cl⁻, and Na⁺, are maintained, thereby establishing a relatively constant cell volume (Hoffmann and Simonsen, 1989; Lande et al., 1995).

In more detail, the transmembrane gradient of sodium and potassium ions is a basic property of mammalian cells with a high concentration of potassium ions inside the cell and a high concentration of sodium ions outside the cell. The sodium potassium pump is one of the major ion pumps of the eukaryotic cell membrane, which is electrogenic and contributes to establishing and maintaining the respective sodium and potassium ionic gradient across the membrane (Kaplan, 1978). The pump uses about 30% of the cell's energy and is one of the major energy-consuming processes of the cells. Many basic biochemical processes are coupled to the electrochemical gradient of sodium ions, such as, e.g. the Na/Ca exchanger or the amino acid transport into cells (Morth et al., 2011). The concentrations of sodium and potassium ions outside a cell are, therefore, parameters of paramount importance that influence the gradient of these ions across the membrane and the basic status of the cell (Paine et al., 1981).

Extracellular Ca²⁺ and Mg²⁺ both have effects on metabolic parameters and macromolecular synthesis in cultured eukaryotic cells. Because of its intracellular concentration and its role in macromolecular synthesis and intracellular transphosphorylation reactions, Mg²⁺ has been suggested as a possible regulator of the positive pleiotropic response, a coordinated array of reactions associated with the initiation of DNA synthesis and multiplication in cultured eukaryotic cells (Achs & Garfinkel, 1973; Lostroh & Krahl, 1974; Rubin, 1975; Rubin, 1978). Ca²⁺, on the other hand, has been suggested as a short-term regulator of cell growth and function, largely based on its tightly regulated low cytoplasmic concentration (Rasmussen et al., 1972; Rebhun, 1977).

Calcium is an abundant bivalent earth metal that exists in many solid inorganic forms in nature. Calcium is an ionically stable bivalent cation with both beneficial and toxic properties in cell culture media (Clapham, 1995). Calcium is involved with a wide range of vital cell functions, including enzyme activities, attachment, motility, tissue morphology, metabolic processes, signal-transduction, replication, and electrochemical responses by specialized cells such as muscle and neural mammalian cells (Borle, 1972).

Since osmolality change in the range of ±50 mmol/kg in the external environment (Baltz, 2012) causes perturbation of intracellular ion homeostasis (because the mechanisms responsible for the generation of osmotic and ionic gradients across the cell membrane cannot be adjusted instantaneously), it is very important to adjust the osmolality of crustacean culture medium in a range similar to the hemolymph.

While most authors used only NaCl at a concentration ranging from 6 to 12 g/L (Chen et al., 1986; Luedeman & Lightner, 1992; Fan & Wang, 2002; Jiang et al., 2005) to increase the total osmolality of culture medium for crustacean cell culture, a few researchers used MgSO₄, MgCl₂, CaCl₂, and KCI at concentrations ranging from 0.9 to 3 g/L (Luedeman & Lightner, 1992; Itami et al., 1999; Lang et al., 2002) for the maintenance of ionic balance and osmotic pressure. However, none of the latter authors disclosed an optimized medium which can support crustacean cells viability and proliferation for a long time. Moreover, although the presence of Mg²⁺, Ca²⁺, and K⁺ -besides Na⁺⁻ is crucial for cells, it is a fact that excessive concentrations of Na⁺, Mg²⁺, Ca²⁺, and K⁺ ions can negatively affect cells in vitro (Schanne et al., 1979; Faber, 1990; Clapham, 1995; Selvaraj & Jayaganesh, 2010). Therefore, it is of paramount importance to have the correct composition of salts in crustacean cells in order to get them in a healthy condition, to be able to culture them for a long time, passage them and stimulate them for proliferation.

The pH of the growth medium is crucial as inappropriate pH may result in poor cell maintenance and growth. The physiological pH of the hemolymph of crustacea ranges from 7.5 to 8.0, which is higher than that of vertebrates (Villena, 2003). In the field of shrimp cell cultures, the pH used in growth media is within the range of 7.0 - 7.5 and stabilized by chemical buffers (phosphate and HEPES buffers) (Fan & Wang, 2002; Gao et al., 2003; Han et al., 2013; Mulford & Austin, 1998; Tapay et al., 1995; Toullec et al., 1996). It varies from 7 (Toullec et al., 1996), through 7.2 (Tong & Miao, 1996; Fan et al., 2002) and 7.4 (Wang et al., 2000), up to 7.5 (Dantas-Lima et al., 2012). Because CO₂ is produced by cultured cells, the pH can easily drop in the medium and chemical buffers are not strong enough to buffer the medium. Furthermore, these chemical buffers may be toxic for cells (Lepe-Zuniga et al., 1987). Therefore, an efficient buffering system that is not toxic is a critical aspect of cell culture. In this context, a CO₂/sodium bicarbonate buffer is preferred because of its ability to keep the pH constant. The pH can affect cell viability, cell migration, and cell proliferation, and it is important to have an efficient buffering system to keep the pH in the desired range for a long time (Kruse et al., 2017).

The optimal temperature for cell cultures depends on the body temperature of the host (Freshney, 2005). It is known that shrimp are cultured in water with a temperature between 25°C and 32°C, and lobsters live in water temperature between 15-18°C. Therefore, most authors have incubated cultures of penaeid tissues at 25-28°C and cultures of lobster tissues at 15-18°C (Han et al., 2013).

Amino acids are a group of organic molecules of which each is comprised of a basic amino group (-NH₂), an acidic carboxyl group (-COOH), and an organic R group (or side chain) that is unique to each amino acid (Gao et al., 2014). Amino acids are building blocks of proteins, as well as the intermediates in metabolism. The amino and the carboxyl groups of amino acids react to form a covalent amide linkage, called a peptide bond. This feature of amino acids allows them to polymerize to form short peptides and long proteins (Holley, 1975). Amino acids can be classified into three groups: essential amino acids, non-essential amino acids and conditional amino acids. Amino acids are commonly used as components in mammalian cell culture media. To prolong the viability of mammalian cells in culture and stimulate growth, supplements of amino acids can be added to media (Wu, 2010).

The genetic composition of cells, their gene expression profiles, the cell cycle, and the environment in which cells are present influence the consumption rates and the metabolic flux of amino acids (Vallee et al., 2014; Fomina-Yadlin et al., 2014; Carrillo-Cocom et al., 2014; Yu et al., 2011; Carinhas et al., 2013). Adding amino acids that are generally consumed does not always lead to the improvement of the cell culture process but could lead to undesired effects (Rouiller et al., 2014; Chen & Harcum, 2006). Therefore, the amino acid content of the cell culture environment should be in ratios dependent on the metabolism and metabolic rates of the specific cell clone and amino acid concentrations must be adjusted to their cellular consumption rates (Salazar et al., 2016). Others have added amino acids in commercial culture medium to culture crustacean cells but none of them resulted in an apparent effect on primary crustacean cell culture (Brody et al., 1989; Itami et al., 1999; Li et al., 2014). Amino acids are different regarding solubility, stability, dissolution kinetics, and interactions with other molecules (Salazar et al., 2016). Since amino acids are important in cellular metabolic pathways and can improve or inhibit metabolism (Frandsen et al., 1987; Li et al., 1995 Schneider et al., 1996; Salazar et al., 2016), it is important to consider amino acids interactions in culture medium. Optimizing medium amino acid composition by adding amino acids on top of the already presented amino acids in the medium without ability to remove or decrease them is not a good strategy since some amino acids can be undesired or have negative interaction with new amino acids. It is crucial to prepare a medium from scratch without using a commercial medium as a basic culture medium to achieve a balanced medium in term of amino acid needs of crustacean cells. The individual effect of different amino acid concentrations, without presence of already presented amino acids in commercial media, was never tested before in crustacean cell culture.

L-alanyl-L-glutamine is a dipeptide substitute for the amino acid L-glutamine. L-alanyl-L-glutamine can be used as a direct substitute for L-glutamine at equimolar concentrations in both adherent and suspension mammalian cell cultures with minimal or no adaptation. L-alanyl-L-glutamine is highly soluble, heat-stable, and improves growth efficiency and performance of mammalian cell culture systems (Huang et al., 2020). L-alanyl-L-glutamine eliminates problems associated with the spontaneous breakdown of L-glutamine into ammonia during incubation, allowing for longer lasting cultures. L-alanyl-L-glutamine supplement is an alternative to L-glutamine, with increased stability that can improve cell health. It has been confirmed in mammalian cell culture that L-alanyl-L-glutamine supplement is suitable for both adherent and suspension culture cells, with no adaptation required. However, there is no information regarding the use of the latter dipeptide for crustacean cell cultures. Compared to L-glutamine, L-alanyl-L-glutamine supplement minimizes toxic ammonia build-up, improves cell viability and growth, and remains stable across a wide range of temperatures (Tritsch & Moore, 1962). Unlike L-glutamine, L-alanyl-L-glutamine supplement does not spontaneously break down to form ammonia. Instead, cells cleave the dipeptide bond to release L-glutamine when needed. This system prevents waste build-up and maintains a fresh supply of L-glutamine during long-term culture. Cultures with lower levels of ammonia show improved cell viability (Atanassov et al., 1998). In addition, culture conditions are more controlled due to the consistent concentration of L-alanyl-L-glutamine that is not possible with L-glutamine. L-alanyl-L-glutamine is room temperature stable and can retain full functionality for 24 months at room temperature, 2 to 8°C, or -5 to -30°C for convenient storage and handling. L-alanyl-L-glutamine is stable at 37°C for a week or more (Zielke et al., 1984; Yaqoob et al., 1997). In shrimp cell culture, attempts have been made with media containing L-glutamine (Dantas-Lima., 2012; Li et al., 2014).

Many vitamins are essential for the growth and proliferation of cells. Vitamins cannot be synthesized in sufficient quantities by cells and are therefore important supplements required in tissue culture. Serum is the major source of vitamins in cell culture. However, media are also enriched with different vitamins making them suitable for a particular cell line (Schnellbaecher et al., 2019). The B group vitamins are crucial for the growth stimulation of mammalian cells, but there is totally no information on the role and need of B group vitamins in crustacean cell culture (Sañudo-Wilhelmy et al., 2014).

Although the concentrations of these components are very low, the availability of vitamins is extremely important for the cells since the cells usually cannot synthesize the vitamins. Vitamins act as co-factors for many enzymes and are essential for their function. Depending on the nature of cultured cells, the absence or presence of certain vitamins in culture may lead to a decrease in cell growth, cell death or loss of productivity (Bram et al., 1980; Lane et al., 1998; Zaken et al., 2001; Ishaque et al., 2002). However, little is known about the effect of vitamins on crustacean cell growth and viability.

Carbohydrates in the form of sugars are the major source of energy. Animal cells (heterotrophs) derive their energy from coupled oxidation-reduction reactions. Glucose is a primary fuel for heterotrophs. Energy derived from glucose is stored in the form of high-energy phosphate bonds in ATP, or other nucleotide triphosphates, and as energy-rich hydrogen atoms associated with the co-enzymes NADP and NAD (Mulukutla et al., 2010). High concentrations of carbohydrates are analogous to a diabetic condition within the cell culture system. This is important because the same processes that can affect cells and molecules in vivo can occur in vitro (Lorenzi et al., 1985; Yki-Järvinen, 1992; Suhaimi et al., 2015). Carbohydrates such as glucose, galactose, maltose, or fructose have been used as a source of energy for mammalian cell cultures (Brent et al., 2009; Leong et al., 2018). Since different cells from different origins and species have variable needs, it is important to use the adjusted concentration of carbohydrates in the medium since unoptimized concentrations of carbohydrates can be toxic (Lorenzi et al., 1985; Hassell et al., 1991; Yki-Järvinen, 1992).

Albumin is a protein in mammal's blood and crustacean hemolymph acting to bind water, salts, free fatty acids, hormones, and vitamins, and transport them between tissues and cells (Yamane et al., 1975; Najafabadi et al., 1992; Qiu et al., 2016). The binding capacity of albumin makes it a suitable remover of toxic substances from the cell culture media (Mendonca et al., 2007; Riley et al., 2008). Lactalbumin is the albumin contained in milk and obtained from whey. Lactalbumin is found in milk of many mammals (Permyakov et al., 2000).

Studies in mammals suggest that certain types of lactalbumin may improve the immune response and increase the levels of glutathione systemically in animals (Francis, 2010). It possesses antiviral, anti-apoptosis and anti-tumor activities in mammalian cells. Albumin content in crustacean hemolymph reflects the body's nutritional and metabolic status (Qiu et al., 2016). Lactalbumin hydrolysate is an enzymatic digest of lactalbumin. Lactalbumin hydrolysate can provide nitrogen, amino acids, vitamins, and carbon in culture media which all can be beneficial for mammalian and insect cells (Maranga et al., 2002; Mendonça et al., 2007). Different cell types have different needs, and it is important that the requirements of crustacean cells in terms of lactalbumin be carefully considered (Schlaeger, 1996; Ikonomou et al., 2003). In crustacean cell cultures, the required concentration of lactalbumin is not known.

Serum is a key component for growing and maintaining cells in culture. Containing a mixture of proteins, hormones, minerals and other growth factors, serum is a nutrient boost for cultured cells. It is added to media as a growth supplement, and specialized forms can be used for different experimental conditions (Barnes et al., 1980; Gstraunthaler, 2003). Nevertheless, one of the greatest concerns over the use of serum in cell culture media is the variability of results in cell-based assays for the activity of a growth factor, drug, or other compounds on cell function. Serum is undefined and not suitable for most cell-based activity assays. The multitude of components found in serum can inhibit the activity of compounds or protein factors in cell-based assays. Moreover, serum is variable in composition and variable in performance on a lot-to-lot basis. Other disadvantages for the use of serum are endotoxin and hemoglobin effects, infectious agents concern, the lack of performance of serum with some cell lines, and ethical concerns (Sato, 1975; Bjare, 1992; Wessman et al., 1999; Gstraunthaler et al., 2013). Ultrafiltered crustacean hemolymph is free of hemocyanin. Hemocyanin is a copper-containing respiratory pigment found in many mollusks (some bivalves, many gastropods, and cephalopods) and arthropods (many crustaceans, some arachnids, and the horseshoe crab, Limulus) (Ellerton et al., 1983). Copper contained hemocyanin is extremely toxic for all types of cells in culture (Aston et al., 2000; Monteiro et al., 2009; Roychoudhury et al., 2010). Ultrafiltered crustacean hemolymph has never been tested in crustacean cell culture.

Cysteine is nutritionally classified as non-essential because it is synthesized in the liver of mammals. They are, however, essential amino acids that must be obtained from an exogenous source in the case of cell culture applications. The amino acid cysteine can be involved in the synthesis of proteins, auto-oxidation, glutathione synthesis, and the synthesis of enzyme CoA (Osman et al., 1997; Atmaca, 2004; Stipanuk et al., 2006). Cysteine serves a very important, though indirect, role of protecting cells from oxidative stress. It is used in the synthesis of the tri-peptide glutathione. Glutathione is the primary aqueous antioxidant involved in blocking lipid peroxidation and dehydroascorbic acid oxidation. In the absence of L-cysteine or its equivalents, glutathione is rapidly depleted and cells will die. L-cysteine also acts as a major source of sulfur in cell culture (Malorni et al., 1995; Atmaca, 2004; Hernández- Ibáñez et al., 2016).

Tyrosine is a polar, non-essential amino acid with phenolic properties. L-tyrosine is a natural amino acid that is incorporated into proteins as directed by the genetic code. The phenolic function makes the tyrosine residue in proteins a principle recipient of reactions such as phosphorylation/dephosphorylation important for cell signaling. Tyrosine is also a precursor of many neurotransmitters and hormones (Kang et al., 2012; Tang et al., 2019).

Biotin is an essential vitamin that is vital for amino acids and energy metabolism, as well as fatty acid synthesis. It is a prosthetic group found in four carboxylase families and facilitates the binding and transfer of carbon dioxide. Fatty acids cannot be synthesized by cells in the absence of biotin. This makes these cells dependent upon external sources of fatty acids, including the palmitoyl fatty acid family (Messmer et al., 1977; Dakshinamurti et al., 1985). As a result of the lack of biotin, cells are not able to convert methionine, isoleucine, homocysteine, or leucine into intermediates in the tricarboxylic acid cycle (TCA) and they cannot convert excess pyruvate into fatty acids. Biotin may play a role in the regulation of transcription and DNA repair (Rodriguez-Melendez and Zempleni, 2003; Dakshinamurti, 2005). Under certain conditions, the enzyme biotinidase appears to function as a biotin transferase and transfer biotin to lysyl residues of histones and polyamines. Histone proteins are important components of chromatin structure that facilitate access to DNA by regulatory and repair proteins. Biotinylation of histones may have a role in silencing of genes and cellular response to DNA damage. Several mitochondrial functions are mediated by polyamines (Stanley et al., 2001; Kuroishi et al., 2011).A proper cell separation method can improve the quality of the cell culture significantly from the very beginning. Different methods that were applied for cell dissociation in crustacean primary cell cultures have been reported with poor results. Adherent cells grow in vitro until they have covered the surface area available, or the medium is depleted of nutrients. At this point, the cells should be subcultured or passaged in order to prevent the culture from dying. To subculture the cells, they need to be brought into suspension. The degree of adhesion varies from cell line to cell line, but in the majority of cases, proteases, e.g. trypsin, are used to release the cells from the flask. However, this may not be appropriate for some cell types where exposure to proteases is harmful or where the enzymes used remove membrane markers/receptors of interest. Toullec (1999) found that the pronase and trypsin could damage crustacean cells while dispase and collagenase IV were less aggressive but weak to dissociate the cells of an explant enzymatically. It was reported that cells of lymphoid organs from *P. japonicus* failed to survive after treatment with trypsin for cell dissociation (Itami et al., 1999). Toxicity of trypsin has also been observed in the primary cell cultures of *P. vannamei* (George & Dhar, 2010). Furthermore, cells can be brought into suspension into a small volume of medium mechanically with the aid of cell scrapers. However, this approach causes mechanical damage to cells. As a result, it is important to establish a reliable method for cell detachment without damaging the cells. Coating the culture surface and targeting the coating substrate instead of cells with enzymes that are not toxic for cells can be a good option for subculturing crustacean cell cultures.

After white spot syndrome virus (WSSV) was firstly detected in Taiwan in 1992, reliable detection and diagnostic techniques were set up. Most of these techniques were based on PCR and histopathology. Cell culture is a basic tool for the study of pathogen-cell interactions, especially for those pathogens that replicate intracellularly, such as viruses (Jiravanichpaisal et al., 2006). For a better understanding of the replication cycle of WSSV and other aquatic viruses, cell cultures are needed. At present, there are no continuous crustacean cell lines available, and therefore, primary cell cultures of crustacea have been used for testing their susceptibility to WSSV. Chen and Kou infected cultured cells from the lymphoid organ of *P. monodon* by monodon-type baculovirus (MBV) in 1989. However, there were only parts of the cell monolayer susceptible to MBV. Tapay et al. set up a 50% tissue culture infectious dose assay protocol for yellow head baculovirus (1995) and non-occluded baculo-like virus based on the primary cell culture from the lymphoid organ of *P. stylirostris* and *P. vannamei* (Tapay, 1995). Kasornchandra and Boonyaratpalin (1998) established a primary cell culture system from the lymphoid organ of *P. monodon,* and this cell culture system was used for titration of white spot syndrome virus (WSSV) from different tissues of infected *P. monodon.* They concluded that the infection of WSSV was dependent on the temperature. Replication was not observed at 4 °C. In the following years, Jose et al. (2012) and Han et al. (2013) proved that the primary cell cultures from the lymphoid organ of P. monodon, *Metapenaeus ensis* and *P. vannamei* were susceptible to WSSV.

In order to replicate viruses in cultured cells, monolayers of healthy cells are essential, therefore, a culture medium should be optimized. This technology can then be used for the diagnosis of viral diseases and to study virus-cell interactions (binding, entry, genome replication, and antiviral responses). Furthermore, these models can be used for the screening of antiviral products.

### Brief description of figures

**Fig. 1****.** Monolayer formation of cells growing out of shrimp lymphoid organ explants at 0, 7, and 14 days of culture in media containing different concentrations of inorganic salts. Arrows show *p-value < 0.05.*
**Fig. 2****.** Monolayer formation of cells growing out of shrimp lymphoid organ explants at 0, 7, and 14 days of culture in media with or without the CO₂/bicarbonate buffer system. No significant difference (*p-value < 0.05*) was observed.
**Fig. 3****.** Monolayer formation of cells growing out of shrimp lymphoid organ explants at 0, 7, and 14 days of culture in media containing different concentrations of amino acids. Arrow shows *p-value < 0.05.*
**Fig. 4****.** Monolayer formation of cells growing out of shrimp lymphoid organ explants at 0, 7, and 14 days of culture in media containing different concentrations of MEM non-essential amino acids stock solution. Arrows show *p-value < 0.05.*
**Fig. 5****.** Monolayer formation of cells growing out of shrimp lymphoid organ explants at 0, 7, and 14 days of culture in media containing different concentrations of glutamine. Arrows show *p-value* < *0.05.*
**Fig. 6****.** Monolayer formation of cells growing out of shrimp lymphoid organ explants at 0, 7, and 14 days of culture in media containing different concentrations of vitamins. Arrows show *p-value < 0.05.*
**Fig. 7****.** Monolayer formation of cells growing out of shrimp lymphoid organ explants at 0, 7, and 14 days of culture in different media contain various concentrations of glucose. Arrow shows *p-value < 0.05.*
**Fig. 8****.** Monolayer formation of cells growing out of shrimp lymphoid organ explants at 0, 7, and 14 days of culture in different media contain various concentrations of lactalbumin. Arrows show *p-value* < *0.05.*
**Fig. 9****.** Monolayer formation of cells growing out of shrimp lymphoid organ explants at 0, 7, and 14 days of culture in different media contain fetal calf serum or shrimp hemolymph. No significant difference (*p-value* < *0.05*) was observed.
**Fig. 10****.** Shrimp lymphoid organ cells are dividing 14 days after culture, A: light microscopy; B: Hoechst-stained cells under fluorescence microscope. Arrows show cells in mitosis.
**Fig. 11****.** Number, monolayer formation, percentage of mitotic cells, and viability of cells growing out of shrimp lymphoid organ explants at 0, 7, and 14 days of culture in different media contain various concentrations of L-cysteine.
**Fig. 12****.** Number, monolayer formation, percentage of mitotic cells, and viability of cells growing out of shrimp lymphoid organ explants at 0, 7, and 14 days of culture in different media contain various concentrations of L-tyrosine. Arrows show *p-value < 0.05.*
**Fig. 13****.** Number, monolayer formation, percentage of mitotic cells, and viability of cells growing out of shrimp lymphoid organ explants at 0, 7, and 14 days of culture in different media contain various concentrations of biotin. Arrows show *p-value < 0.05.*
**Fig. 14****.** Number, monolayer formation, percentage of mitotic cells, and viability of cells growing out of shrimp lymphoid organ explants at 0, 7, and 14 days of culture in four different culture media. Arrow shows *p-value < 0.05.*
**Fig. 15****.** Number, monolayer formation, and viability of cells growing out of shrimp ovary explants at 0, 7, and 14 days of culture.
**Fig. 16****.** Shrimp embryo in culture is developing through different embryonic stages cultured in this invention as culture medium.
**Fig. 17****.** Number, monolayer formation, and viability of cells growing out of lobster hematopoietic explants at 0, 7, and 14 days of culture.
**Fig. 18****.** Shrimp lymphoid organ cells' viability after subculturing at three time points. 1: first subculture; 2: second subculture (3 days after first subculture); 3 third subculture (3 days after second subculture).
**Fig. 19****.** Shrimp lymphoid organ cell culture on gelatin coated surface, 24 hours after first subculture using collagenase type IV.
**Fig. 20****.** Percentage of infected cells in lymphoid organ cells at 0, 24, and 48 hours post inoculation with WSSV Thai 1.

### Description of invention

The present invention thus discloses a cell culture medium comprising: 1) a specific salt composition, in part responsible for the correct osmolality and pH, 2) a specific amino acid and vitamin composition, 3) energy sources such as glucose, and 4) crustacean hemolymph. The present invention further discloses that said cell culture medium is capable to keep cells obtained from shrimp and lobster viable for long periods of time even after passaging the cells into subcultures and induces said cells to proliferate.

The term 'a cell culture medium' relates to a composition which usually comprises multiple compounds and which can be used to keep cells, tissues or organs -which are removed from a crustacean- alive into an artificial environment. Said culture medium can not only be used to keep cells alive but it can also be used to induce proliferation of said cells.

The present invention relates in first instance to a cell culture medium comprising: 1) an osmolality of 835 +/- 55 mmol/kg, 2) a pH ranging between 7.2 and 7.4, 3) the amino acids: glycine, L-alanine, L-aspartic acid, L-asparagine, L-glutamic acid, L-alanyl-L-glutamine, L-proline, and L-serine, 4) the vitamins: choline chloride, calcium pantothenate, folic acid, nicotinamide, pyridoxal hydrochloride, riboflavin, thiamine hydrochloride and inositol, and 5) the supplements: glucose, lactalbumin, and ultrafiltered crustacean hemolymph.

In addition, the present invention relates to a cell culture medium as defined above which further comprises the amino acids L-cysteine and L-tyrosine, and the vitamin biotin.

The term 'an osmolality of 835 +/- 55 mmol/kg' relates to an osmolality ranging from 780 to 890 mmol/kg and can be -for example- equal to 780, 785, 790, 795, 800, 805, 810, 815, 820, 825, 830, 835, 840, 845, 850, 855, 860, 865, 870, 875, 880, 885 or 890 mmol/kg.

The term 'a pH ranging between 7.2 and 7.4' relates- for example- to a pH equal to 7.2, 7.3 or 7.4.

The terms 'the amino acids: glycine, L-alanine, L-aspartic acid, L-asparagine, L-glutamic acid, L-alanyl-L-glutamine, L-proline, L-serine, L-cysteine and L-tyrosine' relates to 9 well-known amino acids and 1 dipeptide (L-alanyl-L-glutamine) which is a dipeptide substitute for the amino acid L-glutamine.

The terms 'the vitamins: choline chloride, calcium pantothenate, folic acid, nicotinamide, pyridoxal hydrochloride, riboflavin, thiamine hydrochloride, inositol and biotin' relate to 9 well-known vitamins.

The terms 'the supplements: glucose, lactalbumin, and ultrafiltered crustacean hemolymph' relate to the well-known compound glucose which is a major source of energy for the cells, the well-known compound lactalbumin which is found in milk of many mammals, and hemolymph collected from a crustacean which is rendered free of hemocyanin via ultrafiltration.

More specifically, the present invention relates to a cell culture medium as described above wherein said osmolality is determined by the addition of the salts: sodium chloride, magnesium sulphate, magnesium chloride.6H₂O, calcium chloride.2H₂0 and potassium chloride. The latter 5 salts are well-known compounds.

More specifically, the present invention relates to a cell culture medium as described above wherein said pH is determined by the addition of sodium bicarbonate and the presence of CO₂ in the air. The well-known CO₂/sodium bicarbonate buffer is indeed preferred and keeps the pH constant (i.e. a pH equal to 7.2, 7.3 or 7.4).

Even more specifically, the present invention relates to a cell culture medium as described above wherein said salts have the following concentrations: 8.27 +/- 0.8 g/L sodium chloride, 0.065 g/L magnesium sulphate, 0.064 g/L magnesium chloride.6H₂O, 0.27 g/L calcium chloride.2H₂0, 0.16 g/L potassium chloride, 0.00942 g/L monopotassium phosphate and 0.0298 g/L disodium phosphate. The terms 8.27 +/- 0.8 g/L sodium chloride refer to a range between 7.47 and 9.07 g/L sodium chloride. The concentrations sodium chloride can thus be for example equal to 7.47, 7.57, 7.67, 7.77, 7.87, 7.97, 8.07, 8.17, 8.27, 8.37, 8.47, 8.57, 8.67, 8.77, 8.87, 8.97 or 9.07 g/L.

More specifically, the present invention relates to a cell culture medium as defined above wherein said pH is determined by the addition of the following concentrations of sodium bicarbonate and CO₂: 2 g/L sodium bicarbonate and the presence of 5% CO₂ in the air.

More specifically, the present invention relates to a cell culture medium as defined above comprising the following concentrations of amino acids, vitamins, and supplements: 3.75-37.5 mg/L glycine, 4.45-44.5 mg/L L-alanine, 6.65-66.5 mg/L L-aspartic acid, 6.6-66 mg/L L-asparagine,7.35-73.5 mg/L L-glutamic acid, 217.22-651.66 mg/L L-alanyl-L-glutamine, 5.75-57.5 mg/L L-proline and 5.25-52.5 mg/L L-serine, 10.0-50.0 mg/L L-cysteine, 10.0-50.0 mg/L L-tyrosine, 1.0-2.0 mg/L choline chloride, 1.0-2.0 mg/L calcium pantothenate, 1.0-2.0 mg/L folic acid, 1.0-2.0 mg/L nicotinamide, 1.0-2.0 mg/L pyridoxal hydrochloride, 0.1-0.2 mg/L riboflavin, 1.0-2.0 mg/L thiamine hydrochloride and 2-4 mg/L inositol, 0.02-0.2 mg/L biotin, 1-2 g/L glucose, 1-2 g/L lactalbumin, and 5% of final volume ultrafiltered crustacean hemolymph. The terms 'a concentration of 3.75-37.5 mg/L' refer to a range of concentrations between 3.75 and 37.5 mg/L and can be, for example, equal to 3.75, 5, 11.25, 13.35, 15, 15.75 17.25 19.80, 19.95, 20, 22.05 25, 30, or 37.5 mg/L. The terms 'a concentration of 4.45-44.5 mg/L' refer to a range of concentrations between 4.45 and 44.5 mg/L and can be, for example, equal to 4.45, 5, ,10, 11.25, 13.35, 15, 15.75 17.25 19.80, 19.95, 20, 22.05 25, 30, 35, 37.5, 40, 42.5, or 44.5 mg/L. The terms 'a concentration of 6.65-66.5 mg/L' refer to a range of concentrations between 6.65 and 66.5 mg/L and can be, for example, equal to 6.65, 7, 8, 10, 11.25, 13.35, 15, 15.75 17.25 19.80, 19.95, 20, 22.05 25, 30, 35, 37.5, 40, 42.5, 45, 50, 55, 59.5, 60, 62.5, or 66.5 mg/L. The terms 'a concentration of 6.6-66 mg/L' refer to a range of concentrations between 6 and 66 mg/L and can be, for example, equal to 6, 7, 8, 10, 11.25, 13.35, 15, 15.75 17.25 19.80, 19.95, 20, 22.05 25, 30, 35, 37.5, 40, 42.5, 45, 50, 55, 59.5, 60, 62.5, or 66 mg/L. The terms 'a concentration of 7.35-73.5 mg/L' refer to a range of concentrations between 7.35 and 73.5 mg/L and can be, for example, equal to 7.35, 7.5, 8, 10, 11.25, 13.35, 15, 15.75 17.25 19.80, 19.95, 20, 22.05 25, 30, 35, 37.5, 40, 42.5, 45, 50, 55, 59.5, 60, 62.5, 65,70, 71.25, 73 or 73.5 mg/L. The terms 'a concentration of 5.75-57.5 mg/L' refer to a range of concentrations between 5.75 and 57.5 mg/L and can be, for example, equal to 5.75, 5.9, 6, 6.5, 7, 10, 11.25, 13.35, 15, 15.75 17.25 19.80, 19.95, 20, 22.05 25, 30, 35, 37.5, 40, 42.5, 45, 50, 55, or 57.5 mg/L. The terms 'a concentration of 5.25-52.5 mg/L' refer to a range of concentrations between 5.25 and 52.5 mg/L and can be, for example, equal to 5.25, 5.5, 6, 6.5, 7, 10, 11.25, 13.35, 15, 15.75 17.25 19.80, 19.95, 20, 22.05 25, 30, 35, 37.5, 40, 42.5, 45, 50, 52, or 52.5 mg/L. Similarly, 'a concentration of 1.0-2.0 mg/L' refer to a range of concentrations between 1.0 and 2.0 mg/L and can be, for example, equal to 1, 1.1, 1.2, 1.3, 1.4, 1.5, 1.6, 1.7, 1.8, 1.9 or 2.0 mg/L. The terms '217.22-651.66 mg/L L-alanyl-L-glutamine' refer to a range of concentrations between 217.22 and 651.66 mg/L L-alanyl-L-glutamine and can be -for example- equal to 217.22, 267.22, 317. 22, 367.22, 417, 22, 434.44, 467.22, 517.22, 567.22, 617.22 or 651.77 mg/L L-alanyl-L-glutamine. The terms '10.0-50.0 mg/L L-cysteine' refer to a range of concentrations between 10.0 and 50.0 mg/L L-cysteine and can be -for example- equal to 10, 15, 20, 25, 30, 35, 40, 45 or 50 mg/L L-cysteine. Similarly,'a concentration of 10.0-50.0 mg/L L-tyrosine' refer to a range of concentrations between 10.0 and 50.0 mg/L L-tyrosine and can be -for example-equal to 10, 15, 20, 25, 30, 35, 40, 45 or 50 mg/L L-tyrosine. The terms '0.1-0.2 mg/L riboflavin' relate to a range between 0.1 and 0.2 and can be -for example- equal to 0.1, 0.15, or 0.2 mg/L riboflavin. Similarly, the terms '2-4 mg/L inositol' refer to a range between 2 and 4 mg/L inositol and can be -for example- equal to 2, 3, or 4 mg/L inositol. The terms '1-2 g/L' relate to a range between 1 and 2 g/L and can be for example equal to 1, 1.5, or 2 g/L. Similarly, 'a concentration of 0.02-0.2 mg/L biotin' refers to a range between 0.02-0.2 mg/L biotin and can be -for example- equal to 0.02, 0.04, 0.06, 0.08, 0.1 or 0.2 mg/L biotin.

Even more specifically, the present invention relates to a cell culture medium as described above comprising the following concentrations of amino acids, vitamins, and supplements: 11.25 mg/L glycine, 13.35 mg/L L-alanine, 19.95 mg/L L-aspartic acid, 19.80 mg/L L-asparagine,22.05 mg/L L-glutamic acid, 434.44 mg/L L-alanyl-L-glutamine, 17.25 mg/L L-proline, 15.75 mg/L L-serine, 30.0 mg/L L-cysteine, 30.0 mg/L L-tyrosine, 1.57 mg/L choline chloride, 1.57 mg/L calcium pantothenate, 1.57 mg/L folic acid, 1.57 mg/L nicotinamide, 1.57 mg/L pyridoxal hydrochloride, 0.15 mg/L riboflavin, 1.57 mg/L thiamine hydrochloride and 3.14 mg/L inositol, 0.11 mg/L biotin, 1.5 g/L glucose, 1.5 g/L lactalbumin, and 5% of final volume ultrafiltered crustacean hemolymph.

Furthermore, the present invention relates to a cell culture medium as described above which further comprises at least one of the following compounds: penicillin, streptomycin, gentamicin, amphotericin B.

Penicillin, streptomycin, gentamicin and amphotericin B are well-known antibiotics.

More specifically, the present invention relates to a cell culture medium as described above which further comprises the following concentrations of compounds: 100000 U/L penicillin, 100 mg/L streptomycin, 50 mg/L gentamicin and 0.25 mg/L amphotericin B.

Moreover, the present invention relates to the usage of a cell culture medium as described above to maintain the viability of cells and/or to proliferate cells taken from a crustacean in vitro.

More specifically, the present invention relates to a usage as described above wherein said cells taken from a crustacean are cells or explants taken from a lymphoid organ, an embryo, an ovary, or a hematopoietic tissue of said crustacean.

The term 'lymphoid organ' (LO) relates to a crustacean organ which is situated -in shrimp- ventral to the stomach and slightly dorso-anterior to the ventral hepatopancreas (Duangsuwan et al., 2008b). The position of the lymphoid organ differs slightly between the male and the female shrimp. The LO lies between the hepatopancreas and stomach in male prawn and between the ovary and the hepatopancreas in female prawn. Sex and gonad maturation may also contribute to the differences in the LO position. The ovary seems to press this organ onto the upper part of the hepatopancreas (Rusaini & Owens, 2010).

The reproductive system of female -for example- penaeid shrimp consists of paired ovaries, oviducts and a single thelycum. The ovaries are partly fused. bilaterally symmetrical bodies extending in the mature animal for nearly its entire length, usually from the cardiac region of the stomach to the telson (Robertson et al., 1993).

The term 'embryos' relates to different stages; from zygote to 2 cells, 4 cells, blastula, gastrula, limb bud embryo and larva in a membrane. After hatching from the membrane, six nauplius stages, three zoea stages, three mysis stages and post larvae stages are described before it becomes a juvenile shrimp (Toullec et al., 1996; Fan et al., 2002). The hematopoietic tissue of the American lobster is a thin (40-800 pm thick) layer of tissue loosely bound to the dorsal surface of the foregut. It is covered by an incomplete layer of loose connective tissue and contains muscle fibers, blood vessels, and ovoid lobules containing stem cells and maturing hemocytes. The lobules are most abundant over the dorsal surface of the foregut and become gradually replaced by connective tissue and striated muscle fibers towards the anterior and lateral margins of the foregut (Martin et al., 1993; Shields et al., 2014). In the majority of penaeid shrimp, the hematopoietic tissue consists of densely packed lobules and mainly covers the dorsal and dorsolateral sides of the stomach or foregut (Truth, 1980). Besides the epigastric region, the hematopoietic tissue of penaeid shrimp is situated in the maxillipeds (Thomas & Lightner, 1988).

Furthermore, the present invention relates to a usage as described above wherein said crustacean is a shrimp or a lobster.

Non-limiting examples of shrimp are penaeid shrimp such as *Penaeus monodon* (Hsu et al., 1995), *P*. *vannamei, P. japonicus* (Itami et al., 1999), *P. stylirostris* (Shike et al., 2000), *P. chinensis, P. indicus, P. merguiensis, P. orientates, P. aetecus* and *P. ensis.* A non-limiting example of a lobster is *Homarus americanus,* an economically important species (Stepanyan et al., 2004).

Moreover, the present invention further describes a method to obtain a subculture from the proliferating cells obtained via the usage of a cell culture medium as described above comprising: 1) washing said proliferating cells using a buffered washing solution to remove bivalent ions that are crucial for cell attachment, and 2) detaching said proliferating cells from a plate coated with gelatin or collagen as coating material with a gelatinase or collagenase enzyme capable of cleaving said coating material.

A proper cell separation method can improve the quality of the cell culture significantly from the very beginning. Adherent cells will grow in vitro until they have covered the surface area available, or the medium is depleted of nutrients. At this point, the cells should be subcultured or passaged in order to prevent the culture from dying. Coating the culture surface and targeting the coating substrate instead of cells with enzymes that are not toxic for cells can be a good option for subculturing crustacean cell cultures.

More specifically, the present invention relates to a method as described above wherein said coating material is collagen IV, wherein said enzyme is collagenase IV and wherein said buffered washing solution comprises: a pH ranging between 7.2 and 7.4, an osmolality of 835±55 mmol/kg, 371.9±25 mM NaCl, 8 mM Na₂HPO₄, 2 mM KH₂PO₄, and 2.7 mM KCl. The term 'an osmolality of 835 +/- 55 mmol/kg' relates to an osmolality ranging from 780 to 890 mmol/kg and can be -for example- equal to 780, 785, 790, 795, 800, 805, 810, 815, 820, 825, 830, 835, 840, 845, 850, 855, 860, 865, 870, 875, 880, 885 or 890 mmol/kg. The term '371.9±25 mM NaCl' relates to a concentration ranging from 346.9 to 396.9 mM NaCl and can be -for example- equal to 346.9, 355, 365, 371.9, 375, 385, 395 or 396.9mM NaCl.

Collagen IV, collagenase IV, NaCl, Na₂HPO₄, KH₂PO₄ and KCI are well-known compounds.

The present invention further describes the usage of the viable and proliferating cells obtainable by a usage as described above and/or a method as described above as host cells for crustacean pathogens.

Non-limiting examples of crustacean pathogens are white spot syndrome virus (WSSV), infectious hypodermal and hematopoeitic necrosis virus (IHHNV), monodon baculovirus (MBV), taura syndrome virus (TSV), yellow head virus (YHV), gill associated virus (GAV), Vibrio campbelli, Vibrio harveyi, and Vibrio parahaemolyticus.

### Examples

### Material and methods

### 1. Experimental animals

*Litopenaeus vannamei* post-larvae which were imported from Syaqua Siam Co. Ltd. (Thailand) were certified to be specific pathogen-free for WSSV, TSV, YHV, IHHNV and IMNV. After arrival, the post-larvae were stocked in a recirculating aquaculture system with 3.5% salinity at Imaqua, Gent, Belgium. These shrimp were reared with commercial pelleted feed. After 4 months, shrimp in premolt stage weighing 20 ± 2 g were used for the collection of lymphoid organ, ovary, and embryos. American lobsters (*Homarus americanus*) weighing 600 ± 25 g were purchased at a local store and stocked in sea water with 3.3% salinity. Lobsters were kept for 1 week at the Laboratory of Virology, Ghent University, Belgium at 20°C before using their hematopoietic organ for cell culture.

### 2. Culture medium preparation

Two times concentrated L15 medium and lab made culture media were prepared by diluting powders and liquids in ultra-pure water. All the liquids such as amino acids and vitamins stock solutions, sodium pyruvate, grace insect medium (Gibco), fetal calf serum (Sigma-Aldrich), penicillin (Gibco), streptomycin (Gibco), and amphotericin B (Gibco) were available commercially. Leibovitz's medium (Sigma-Aldrich), inorganic salts (all from Sigma-Aldrich), sodium bicarbonate (Sigma-Aldrich), lactalbumin (Fluka), galactose (Merck), glucose (Merck), and phenol red (Merck) were available commercially as powder. Stock salt solutions have been made by weighing all the components and transferring them to a bottle followed by mixing with ultra-pure water. After preparing the medium, osmolality was measured using a Fiske 210 osmometer and adjusted to the desired value. All the culture media and stock solutions were sterilized using a 0.2µm filter. Different media containing 2X L15 medium with various concentrations of components has been prepared and used as basal medium for inorganic salts and osmolality and buffering system adjustment experiments. 2X L15 medium has also been used as basal medium for adjusting the amino acid composition. For some experiments a lab made medium based on ultra-pure (UP) water and different salts and amino acids have been used. To evaluate the function of the finally obtained medium (Table 9), a comparison with previous studies has been done. For this end, reference media were prepared as shown in Table 1.

**Table 1. Reference culture media composition**

| **Composition** | **Osmolality** | **Reference** |
|---|---|---|
| 68.5% 2X L15 | 900 | Li et al., 2014 |
| 20% fetal bovine serum | | |
| 10% Chen's salt (Li et al, 2014) | | |
| 1% penicillin/streptomycin (100000U/L | | |
| penicillin, 100mg/L streptomycin) | | |
| 0.5% gentamicin (50 mg/L), | | |
| 0.25 mg/L amphotericin B | | |
| 84% Grace's Insect medium | 750 | Luedeman & Lightner, 1992 |
| 10% fetal bovine serum | | |
| 1% magnesium chloride (2 Molar) | | |
| 1% sodium chloride (5 Molar) | | |
| 1% proline (2 mg/ml) | | |
| 1% sodium bicarbonate (40 mg/ml) | | |
| 1% Penicillin/streptomycin (10000U/ml | | |
| Penicillin, 10000 µg/ml streptomycin) | | |
| 1% amphotericin B (10 | | |
| µg/ml) | | |
| 50% 2X L-15 medium | 720±10 | Chen et al., 1986 |
| 10% fetal bovine serum | | |
| 30% muscle extract | | |
| 0.006g/ml NaCl | | |
| 10% lobster or grass prawn hemolymph | | |

### 2.1. Inorganic salts and osmolality adjustment

To achieve a balanced shrimp cell culture medium in terms of inorganic salts and osmolality, first 16 salt solutions (salt solution 1 to salt solution 16) were prepared (Table 2). Salt solutions have been made by weighing all the components mentioned in Table 2 and transferring them to a bottle followed by mixing with one liter ultra-pure water. Then 16 different media were prepared with each medium containing 78.5% 2X L15 medium, 10% fetal calf serum, 10% stock salt solution (salt solution 1 to salt solution 16), 1% penicillin/streptomycin (100000U/L penicillin, 100mg/L streptomycin), 0.5% gentamicin (50 mg/L gentamicin), and 0.0025% amphotericin B (0.25 mg/L amphotericin B). Salt solution 1 known as Chen's salt has been used before by several researcher for culturing crustacean cells (Li et al., 2014), so the medium containing the salt solution 1 has been considered as reference. The osmolality of the medium containing 10% salt solution 1 was 927 mmol/kg while the osmolality of media containing 10% salt solutions 2 to 16 (Table 2) adjusted to 780 mmol/kg, which is the same as the hemolymph of *Litopenaeus Vannamei* shrimp. Shrimp lymphoid organ explants were cultured in different media and incubated at 27°C, 0% CO2 (pH 7.2-7.4). Monolayer formation of cells growing out of the shrimp lymphoid organ explant were analyzed at 0, 7, and 14 days of culture.

**Table 2. Composition of different inorganic stock salt solutions**

| **Stock salt solutions** | **Concentration of inorganic salts (g/L) in stock salt solutions** | | | | | **Final medium osmolality (mmol/kg) when stock salt solution added as 10% of complete 2X L15 medium (v/v)** |
|---|---|---|---|---|---|---|
| | **NaCl** | **MgSO₄.7H₂O** | **MgCl₂.6H₂O** | **CaCl₂.2H₂O** | **KCl** | |
| Salt solution 1 | 102.4 | 16.7 | 11.8 | 5.1 | 1.8 | 927 |
| Salt solution 2 | 71.12 | 16.7 | 11.8 | 5.1 | 1.8 | 780 |
| Salt solution 3 | 72.91 | - | - | - | - | 780 |
| Salt solution 4 | 72.57 | 0.5 | 0.5 | - | - | 780 |
| Salt solution 5 | 71.42 | - | - | 2.5 | - | 780 |
| Salt solution 6 | 72.12 | - | - | - | 1 | 780 |
| Salt solution 7 | 71.08 | 0.5 | 0.5 | 2.5 | - | 780 |
| Salt solution 8 | 71.79 | 0.5 | 0.5 | - | 1 | 780 |
| Salt solution 9 | 70.63 | - | - | 2.5 | 1 | 780 |
| Salt solution 10 | 70.2 | 0.5 | 0.5 | 2.5 | 1 | 780 |
| Salt solution 11 | 67.31 | 5 | 5 | 2.5 | 1 | 780 |
| Salt solution 12 | 37.35 | 50 | 50 | 2.5 | 1 | 780 |
| Salt solution 13 | 71.64 | 0.5 | 0.5 | 0.25 | 1 | 780 |
| Salt solution 14 | 56.89 | 0.5 | 0.5 | 25 | 1 | 780 |
| Salt solution 15 | 71.01 | 0.5 | 0.5 | 2.5 | 0.1 | 780 |
| Salt solution 16 | 63.25 | 0.5 | 0.5 | 2.5 | 10 | 780 |

### 2.2. Buffering system adjustment

L15 medium lacks a strong buffering system and is designed to support growth of cells in the absence of CO₂. Since regulating pH is critical for optimal culture conditions, a natural buffering system by using NaHCO₃ and CO₂ has been developed to evaluate the effect of the buffering system on the cells' growth. The needed amount of NaHCO₃ was calculated using the Henderson-Hasselbalch equation. The Henderson-Hasselbalch equation describes the relationship of pH as a measure of acidity with the acid dissociation constant (pKa), in biological and chemical systems. The equation is especially useful for estimating the pH of a buffer solution and finding the equilibrium pH in acid-base reactions. Two different media were prepared and the effect of 2X L15 medium containing 2g/L of NaHCO₃ in the presence of 5% CO₂ was compared with 2X L15 medium without CO₂/NaHCO₃ in the environment. The pH of the medium was measured regularly to be sure that the pH was at 7.2-7.4. Both media were supplemented with 10% salt solution 10 (v/v) (Table 2), 10% fetal calf serum (v/v), 2000 mg/L sodium bicarbonate (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B). Shrimp lymphoid organ explants were cultured in different media at 27°C. The formation of a monolayer of cells growing out of the shrimp lymphoid organ explant were analyzed at 0, 7, and 14 days of culture.

### 2.3. Amino acid adjustment

It is important to consider the amino acid composition in culture medium. Optimizing the amino acid composition by adding amino acids on top of the already present amino acids in the medium without the ability to remove or decrease them is not a good strategy since some amino acids can be undesired or have negative interaction with new amino acids. It is crucial to prepare a medium from scratch to achieve a balanced medium in terms of amino acid needs of crustacean cells. In this experiment, the effect of different amino acid concentrations has been tested. To prepare the media containing different concentrations of amino acids, amino acid mixtures, including MEM amino acids solution (50X) (Thermo Fisher Scientific), MEM non-essential amino acids solution (100X) (Thermo Fisher Scientific), and RPMI 1640 amino acids solution (50X) (Thermo Fisher Scientific) have been used (Table 3).

11 different media (medium 1 to medium 11) containing none, one, or a combination of stock amino acids solutions were prepared as shown in Table 6. Medium 1 contained none of the stock amino acid solutions and was prepared by supplementing 2X L15 medium with 10% salt solution 10 (v/v) (Table 5), 10% fetal calf serum (v/v) (), 2000 mg/L sodium bicarbonate (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Media 2, 3, and 4 contained just one of the following amino acid mixtures: MEM amino acids solution (50X), MEM non-essential amino acids solution (100X), or RPMI 1640 amino acids solution (50X). Media 2, 3, and 4 were prepared by making an ultra-pure water based solution containing an amino acids solution (Table 6), 10% salt solution 17 (v/v) (salt solution 17 consists of salt solution 10 with additional inorganic salts (12.56 g/L NaCl, 0.15 g/L MgSO₄.7H₂O, 0.14 g/L MgCl₂.6H₂O, 0.21 g/L CaCl₂.2H₂O, 0.62 g/L KCl, 0.094 g/L KH₂PO₄, and 0.298 g/L Na₂HPO₄) giving the final salt composition of: 82.76 g/L NaCl, 0.65 g/L MgSO₄.7H₂O, 0.64 g/L MgCl₂.6H₂O, 2.71 g/L CaCl₂.2H₂O, 1.62 g/L KCl, 0.094 g/L KH₂PO₄, and 0.298 g/L Na₂HPO₄) (Table 5), 1.57% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Media 5, 6, and 7 contained just one of the following amino acid mixtures: MEM amino acids solution (50X), MEM non-essential amino acids solution (100X), or RPMI 1640 amino acids solution (50X). Media 5, 6, and 7 were prepared by supplementing 2X L15 medium with an amino acids solution (Table 6), in addition to 10% salt solution 10 (v/v) (Table 5), 10% fetal calf serum (v/v), 2000 mg/L sodium bicarbonate (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Media 8, 9, 10, and 11 contained a combination of the following amino acid mixtures: MEM amino acids solution (50X), MEM non-essential amino acids solution (100X), and RPMI 1640 amino acids solution (50X). Media 8, 9, 10, and 11 were prepared by making an ultra-pure water based solution containing a combination of an amino acids solution (Table 6), 10% salt solution 17 (v/v) (Table 5), 1.57% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

The composition of all the media were the same except in terms of amino acids (Table 7). Shrimp lymphoid organ explants were cultured in different media and incubated at 27°C, 5% CO₂ (pH 7.2-7.4). The formation of a monolayer of cells growing out of the shrimp lymphoid organ explants were analyzed at 0, 7, and 14 days of culture.

**Table 3. Composition of different amino acid solutions**

| **Concentration (mg/L) of amino acids in amino acid stock solutions** | | | |
|---|---|---|---|
| **Amino acids** | **Amino acid stock solution 1: MEM amino acids (50X)** | **Amino acid stock solution 2: MEM non-essential amino acids (100X)** | **Amino acid stock solution 3: RPMI 1640 amino acids (50X)** |
| glycine | - | 750 | 500 |
| alanine | - | 890 | - |
| valine | 2340 | - | 1000 |
| leucine | 2620 | - | 2500 |
| isoleucine | 2620 | - | 2500 |
| phenylalanine | 1650 | - | 750 |
| proline | - | 1150 | 1000 |
| serine | - | 1050 | 1500 |
| threonine | 2380 | - | 1000 |
| tyrosine | 1800 | - | 1441.5 |
| cysteine | 1200 | - | 3260 |
| methionine | 755 | - | 750 |
| asparagine | - | 1320 | 2500 |
| glutamine | - | - | 15000 |
| tryptophan | 510 | - | 2500 |
| aspartic acid | - | 1330 | 1000 |
| glutamic acid | - | 1470 | 1000 |
| lysine | 3625 | - | 2000 |
| arginine | 6320 | - | 10000 |
| histidine | 2100 | - | 750 |

**Table 4. Composition of the vitamin stock solution**

| **Vitamins** | **Concentration (mg/L) of different vitamins in the vitamin stock solution** |
|---|---|
| | **MEM vitamin solution (100X)** |
| Choline chloride | 100 |
| Calcium pantothenate | 100 |
| Folic Acid | 100 |
| Niacinamide | 100 |
| Pyridoxine hydrochloride | 100 |
| Riboflavin | 10 |
| Thiamine | 100 |
| Inositol | 200 |

**Table 5. Composition of two different inorganic salt stock solutions.**

| **Inorganic salts** | **Concentration (g/L) of different inorganic salts in salt stock solutions** | |
|---|---|---|
| | **Salt solution 10** | **Salt solution 17** |
| NaCl | 70.2 | 82.76 |
| MgSO₄.7H₂O | 0.5 | 0.65 |
| MgCl₂.6H₂O | 0.5 | 0.64 |
| CaCl₂.2H₂O | 2.5 | 2.71 |
| KCl | 1 | 1.62 |
| KH₂PO₄ | - | 0.094 |
| Na₂HPO₄ | - | 0.298 |

**Table 6. Composition of different media containing various concentrations of amino acids for culturing cells growing out of Litopenaeus vannamei shrimp lymphoid organ in order to optimize amino acid concentrations.**

| **Medium components** | | **Concentration (mg/L) or % (v/v) of components in media (M1- M11) containing various concentrations of amino acids prepared in basal medium consisting of either 2X L15 or ultra-pure water (UP)** | | | | | | | | | | |
|---|---|---|---|---|---|---|---|---|---|---|---|---|
| | | **M1** | **M 2** | **M 3** | **M 4** | **M 5** | **M 6** | **M 7** | **M 8** | **M 9** | **M10** | **M11** |
| **Basal medium** | | 2X L15 | UP | UP | UP | 2X L15 | 2X L15 | 2X L15 | UP | UP | UP | UP |
| **Amino acids** | | | | | | | | | | | | |
| | MEM amino acids (50X) | - | 2% | - | - | 2% | - | | 2% | 2% | 2% | - |
| | MEM non-essential amino acids (100X) | - | - | 1.5% | - | - | 1.5% | | 1.5% | - | 1.5% | 1.5% |
| | RPMI 1640 amino acids (50X) | - | - | - | 2% | - | - | 2% | - | 2% | 2% | 2% |

| **Vitamins** | | | | | | | | | | | | |
|---|---|---|---|---|---|---|---|---|---|---|---|---|
| | MEM Vitamin Solution (100X) | - | 1.57 % | 1.57 % | 1.57 % | - | - | | 1.57 % | 1.57 % | 1.57 % | 1.57 % |

| **Inorganic salts** | | | | | | | | | | | | |
|---|---|---|---|---|---|---|---|---|---|---|---|---|
| | Salt solution 10 | 10% | - | - | - | 10% | 10% | 10% | - | - | - | - |
| | Salt solution 17 | - | 10% | 10% | 10% | - | - | | 10% | 10% | 10% | 10% |
| | NaHCO₃ | 2000 | 2000 | 2000 | 2000 | 2000 | 2000 | 2000 | 2000 | 2000 | 2000 | 2000 |

| **Other components** | | | | | | | | | | | | |
|---|---|---|---|---|---|---|---|---|---|---|---|---|
| | D+ Galactose | 1413 | 1413 | 1413 | 1413 | 1413 | 1413 | 1413 | 1413 | 1413 | 1413 | 1413 |
| | Sodium Pyruvate | 863.5 | 863.5 | 863.5 | 863.5 | 863.5 | 863.5 | 863.5 | 863.5 | 863.5 | 863.5 | 863.5 |
| | Phenol Red | 15.7 | 15.7 | 15.7 | 15.7 | 15.7 | 15.7 | 15.7 | 15.7 | 15.7 | 15.7 | 15.7 |
| | Fetal calf serum | 10% | 10% | 10% | 10% | 10% | 10% | 10% | 10% | 10% | 10% | 10% |
| | Penicillin (10000 U/mL)/Streptomy cin (10 mg/mL) | 1% | 1% | 1% | 1% | 1% | 1% | 1% | 1% | 1% | 1% | 1% |
| | Gentamicin (10 mg/ml) | 0.5% | 0.5% | 0.5% | 0.5% | 0.5% | 0.5% | 0.5% | 0.5% | 0.5% | 0.5% | 0.5% |
| | Amphotericin B (10 mg/ml) | 0.25 | 0.25 | 0.25 | 0.25 | 0.25 | 0.25 | 0.25 | 0.25 | 0.25 | 0.25 | 0.25 |
| **Final medium osmolality (mmol/L)** | | 835± 55 | 835± 55 | 835± 55 | 835± 55 | 835± 55 | 835± 55 | 835± 55 | 835± 55 | 835± 55 | 835± 55 | 835± 55 |

**Table 7. Full composition of different media containing various concentrations of amino acids used for culturing cells growing out of Litopenaeus vannamei shrimp lymphoid organ in order to optimize amino acid concentrations (more detailed version of Table 6).**

| **Medium components** | **Concentration (mg/L) or % (v/v) of components in media (M1- M11) containing various concentrations of amino acids prepared in basal medium consisting of either 2X L15 or ultra-pure water (UP)** | | | | | | | | | | |
|---|---|---|---|---|---|---|---|---|---|---|---|
| | **M 1** | **M 2** | **M 3** | **M 4** | **M5** | **M 6** | **M 7** | **M 8** | **M9** | **M 10** | **M 11** |
| **Basal medium** | 2X L15 | UP | UP | UP | 2X L15 | 2X L15 | 2X L15 | UP | UP | UP | UP |
| **Amino acids** | | | | | | | | | | | |
| glycine | 314 | - | 11.2 | 10 | 314 | 325.2 | 324 | 11.2 | 10 | 21.25 | 21.25 |
| alanine | 353.2 | - | 13.3 | - | 353.2 | 366.6 | 353.2 | 13.3 | - | 13.35 | 13.35 |
| valine | 157 | 46.8 | - | 20 | 203.8 | 157 | 177 | 46.8 | 66.8 | 66.8 | 20 |
| leucine | 196.2 | 52.4 | - | 50 | 248.6 | 196.2 | 246.2 | 52.4 | 102.4 | 102.4 | 50 |
| isoleucine | 392.5 | 52.4 | - | 50 | 444.9 | 392.5 | 442.5 | 52.4 | 102.4 | 102.4 | 50 |
| phenylalanine | 196.2 | 33 | - | 15 | 229.2 | 196.2 | 211.2 | 33 | 48 | 48 | 15 |
| proline | - | - | 17.2 | 20 | - | 17.2 | 20 | 17.2 | 20 | 37.25 | 37.25 |
| serine | 314 | - | 15.7 | 30 | 294 | 314 | 344 | 15.7 | 30 | 45.75 | 45.75 |
| threonine | 471 | 47.6 | - | 20 | 518.6 | 471 | 491 | 47.6 | 67.6 | 67.6 | 20 |
| tyrosine | 471 | 36 | - | 29 | 507 | 471 | 500 | 36 | 65 | 65 | 29 |
| cysteine | 188.4 | 24 | - | 65 | 212.4 | 188.4 | 253.4 | 24 | 89 | 89 | 65 |
| methionine | 117.7 | 15.1 | - | 15 | 132.8 | 117.7 | 132.2 | 15.1 | 30.1 | 30.1 | 15 |
| asparagine | 392.5 | - | 19.8 | 50 | 392.5 | 412.3 | 442.5 | 19.8 | 50 | 69.8 | 69.8 |
| glutamine | 471 | - | - | 300 | 471 | 471 | 771 | - | 30 | 30 | 300 |
| tryptophan | 31.4 | 10.2 | - | 5 | 41.6 | 31.4 | 36.4 | 10.2 | 15.2 | 15.2 | 5 |
| aspartic acid | - | - | 19.9 | 20 | - | 19.9 | 20 | 19.9 | 20 | 29.95 | 39.95 |
| glutamic acid | - | - | 22.05 | 20 | - | 22.05 | 20 | 22.05 | 20 | 42.05 | 42.05 |
| lysine | 117.7 | 72.5 | - | 40 | 190.2 | 117.7 | 157.7 | 72.5 | 112.5 | 112.5 | 40 |
| arginine | 785 | 126.4 | - | 200 | 911.4 | 785 | 985 | 126.4 | 326.4 | 326.4 | 200 |
| histidine | 392.5 | 42 | - | 15 | 434.5 | 392.5 | 407.5 | 42 | 57 | 57 | 15 |

| **Vitamins** | | | | | | | | | | | |
|---|---|---|---|---|---|---|---|---|---|---|---|
| Choline chloride | 1.57 | 1.57 | 1.57 | 1.57 | 1.57 | 1.57 | 1.57 | 1.57 | 1.57 | 1.57 | 1.57 |
| Calcium pantothenate | 1.57 | 1.57 | 1.57 | 1.57 | 1.57 | 1.57 | 1.57 | 1.57 | 1.57 | 1.57 | 1.57 |
| Folic Acid | 1.57 | 1.57 | 1.57 | 1.57 | 1.57 | 1.57 | 1.57 | 1.57 | 1.57 | 1.57 | 1.57 |
| Niacinamide | 1.57 | 1.57 | 1.57 | 1.57 | 1.57 | 1.57 | 1.57 | 1.57 | 1.57 | 1.57 | 1.57 |
| Pyridoxine hydrochloride | 1.57 | 1.57 | 1.57 | 1.57 | 1.57 | 1.57 | 1.57 | 1.57 | 1.57 | 1.57 | 1.57 |
| Riboflavin | 0.15 | 0.15 | 0.15 | 0.15 | 0.15 | 0.15 | 0.15 | 0.15 | 0.15 | 0.15 | 0.15 |
| Thiamine | 1.57 | 1.57 | 1.57 | 1.57 | 1.57 | 1.57 | 1.57 | 1.57 | 1.57 | 1.57 | 1.57 |
| Inositol | 3.14 | 3.14 | 3.14 | 3.14 | 3.14 | 3.14 | 3.14 | 3.14 | 3.14 | 3.14 | 3.14 |

| **Inorganic salts** | | | | | | | | | | | |
|---|---|---|---|---|---|---|---|---|---|---|---|
| NaCl | 8276 | 8276 | 8276 | 8276 | 8276 | 8276 | 8276 | 8276 | 8276 | 8276 | 8276 |
| MgSO₄.7H₂O | 65 | 65 | 65 | 65 | 65 | 65 | 65 | 65 | 65 | 65 | 65 |
| MgCl₂.6H₂O | 64 | 64 | 64 | 64 | 64 | 64 | 64 | 64 | 64 | 64 | 64 |
| CaCl₂.2H₂O | 271 | 271 | 271 | 271 | 271 | 271 | 271 | 271 | 271 | 271 | 271 |
| KCl | 162 | 162 | 162 | 162 | 162 | 162 | 162 | 162 | 162 | 162 | 162 |
| KH₂PO₄ | 9.42 | 9.42 | 9.42 | 9.42 | 9.42 | 9.42 | 9.42 | 9.42 | 9.42 | 9.42 | 9.42 |
| Na₂HPO₄ | 29.8 | 29.8 | 29.8 | 29.8 | 29.8 | 29.8 | 29.8 | 29.8 | 29.8 | 29.8 | 29.8 |
| NaHCO₃ | 2000 | 2000 | 2000 | 2000 | 2000 | 2000 | 2000 | 2000 | 2000 | 2000 | 2000 |

| **Other components** | | | | | | | | | | | |
|---|---|---|---|---|---|---|---|---|---|---|---|
| D+ Galactose | 1413 | 1413 | 1413 | 1413 | 1413 | 1413 | 1413 | 1413 | 1413 | 1413 | 1413 |
| Sodium Pyruvate | 863.5 | 863.5 | 863.5 | 863.5 | 863.5 | 863.5 | 863.5 | 863.5 | 863.5 | 863.5 | 863.5 |
| Phenol Red | 15.7 | 15.7 | 15.7 | 15.7 | 15.7 | 15.7 | 15.7 | 15.7 | 15.7 | 15.7 | 15.7 |
| Fetal calf serum | 10% | 10% | 10% | 10% | 10% | 10% | 10% | 10% | 10% | 10% | 10% |
| Penicillin (10000 U/mL)/Streptomyci n (10 mg/mL) | 1% | 1% | 1% | 1% | 1% | 1% | 1% | 1% | 1% | 1% | 1% |
| Gentamicin (10 mg/ml) | 0.5% | 0.5% | 0.5% | 0.5% | 0.5% | 0.5% | 0.5% | 0.5% | 0.5% | 0.5% | 0.5% |
| Amphotericin B (10 mg/ml) | 0.25 | 0.25 | 0.25 | 0.25 | 0.25 | 0.25 | 0.25 | 0.25 | 0.25 | 0.25 | 0.25 |
| **Final medium osmolality (mmol/L)** | 835± 55 | 835± 55 | 835± 55 | 835± 55 | 835± 55 | 835± 55 | 835± 55 | 835± 55 | 835± 55 | 835± 55 | 835± 55 |

### 2.4. L-alanyl-L-glutamine adjustment

L-alanyl-L-glutamine as a source of energy can increase stability and improve cell health in culture. To evaluate the effect of L-alanyl-L-glutamine on shrimp cell culture, an experiment was designed to check the effect of different concentrations of L-alanyl-L-glutamine on shrimp lymphoid organ cells growth. Five testing media containing 0 mM, 1 mM, 2 mM, 3 mM, and 10 mM L-alanyl-L-glutamine (Gibco) were prepared. The basal medium for preparing treatment media was prepared by making an ultra-pure water based solution containing 10% salt solution 17 (v/v) (Table 5), 1.5% MEM non-essential amino acids stock solution (v/v) (Table 3), 1.57% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Shrimp lymphoid organ explants were cultured in different media and incubated at 27°C, 5% CO₂ (pH 7.2-7.4) The formation of a monolayer of cells growing out of the shrimp lymphoid organ explants were analyzed at 0, 7, and 14 days of culture.

### 2.5. Vitamins' adjustment

Since many vitamins are essential for growth and proliferation of cells and they cannot be synthesized in sufficient quantities by cells, vitamin supplementation is required in cell cultures. In order to optimize the vitamin contents of medium, the performance of five media containing 0%, 1%, 1.5%, 2%, and 5% MEM vitamin stock solution (100X) (Thermo Fisher Scientific) were evaluated.

The basal medium for preparing testing media was prepared by making an ultra-pure water based solution containing 10% salt solution 17 (v/v) (Table 5), 1.5% MEM non-essential amino acids stock solution (v/v) (Table 3), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Shrimp lymphoid organ explants cultured in different media and incubated at 27°C, 5% CO₂ (pH 7.2-7.4). Monolayer formation of cells growing out of the shrimp lymphoid organ explant were analyzed at 0, 7, and 14 days of culture.

### 2.6. Glucose supplementation

Glucose as a carbohydrate is an important source of energy for cells in culture. We examined the performance of media containing 0 g/L, 1 g/L, 2 g/L, and 10 g/L glucose concentrations.

The basal medium for preparing treatment media were prepared as described in section 2.5 by making an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Reference medium was prepared by making an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v) (Table 6), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality). Shrimp lymphoid organ explants cultured in different media and incubated at 27°C, 5% CO₂ (pH 7.2-7.4 and 835±55mmol/kg osmolality). The formation of a monolayer of cells growing out of the shrimp lymphoid organ explants were analyzed at 0, 7, and 14 days of culture.

### 2.7. Lactalbumin supplementation

Lactalbumin binds water, salts, free fatty acids, hormones and vitamins, and transports them between tissues and cells. The binding capacity of albumin makes it a suitable remover of toxic substances from the cell culture media. We examined the effect of 0 g/L, 1 g/L, 2 g/L, and 10 g/L lactalbumin concentrations in an ultra-pure water based solution developed in section 2.6 containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 1.5 g/L glucose (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality). Shrimp lymphoid organ explants cultured in different media and incubated at 27°C, 5% CO₂ (pH 7.2-7.4). The formation of a monolayer of cells growing out of the shrimp lymphoid organ explants were analyzed at 0, 7, and 14 days of culture.

### 2.8. Hemolymph supplementation

Serum is a key component for growing and maintaining cells in culture. It is added to media as a growth supplement, and specialized forms can be used for different experimental conditions (Barnes et al., 1980; Gstraunthaler, 2003). Nevertheless, one of the greatest concerns over the use of serum in cell culture media is the variability of results in cell-based assays. Serum is undefined and not suitable for most cell-based activity assays. Ultrafiltered crustacean hemolymph as a crustacean specific source of nutrients can be a good candidate to be used as medium supplement.

Two liter of marine anticoagulant (MA) was prepared by mixing 52.6g sodium chloride, 36 g glucose, 17.6 g tri-sodium citrate (2H₂O), 10 g citric acid (1H₂O), and 7.4 g EDTA with 2 liter of ultra-pure water. The solution was stirred with a magnetic stirrer until all the powder was dissolved. The pH was measured and adjusted to 5.4 with NaOH. The solution was sterilized using a 0.2µm membrane.

Hemolymph was taken from the ventral hemal sinus with a 25-gauge needle and mixed 1:1 using MA. All the material was kept on ice to prevent hemocyte activation. Hemolymph was gently mixed and centrifuged for 10 minutes at 1200 rpm at 4°C to pellet hemocytes. The supernatant was filtered using a 50 kb cut off filter to remove hemocyanin. 50 kb filter membrane was washed two times using 4 ml molecular grade up water by centrifugation at 3300g for 3 minutes at 4°C. The eluted water discarded, and hemolymph added immediately to the column and centrifuged at 3300g for 15 minutes at 4°C. Eluted hemolymph collected and stored at -20°C until use.

Four different media containing 10% fetal calf serum or 1%, 2% or 5% hemolymph were used to evaluate serum/hemolymph supplementation effect on *Litopenaeus Vannamei* shrimp lymphoid organ cell culture monolayer formation.

The medium that was developed in section 2.7 (consisted of an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 2000 mg/L sodium bicarbonate (w/v), 1.5 g/Lglucose (w/v), 1.5 g/L lactalbumin (w/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality)) was used for preparing testing media. Shrimp lymphoid organ explants were cultured in the different media and incubated at 27°C, 5% CO₂ (pH 7.2-7.4 and 835±55mmol/kg osmolality). The formation of a monolayer of cells growing out of the shrimp lymphoid organ explants were analyzed at 0, 7, and 14 days of culture.

### 2.9. L-cysteine supplementation

Cysteine can be involved in protein synthesis, auto-oxidation, synthesis of glutathione, and synthesis of Enzyme CoA. Cysteine serves a very important, though indirect, role of protecting cells from oxidative stress. L-cysteine supplementation of cell culture medium was evaluated in terms of its effects on crustacean cell culture. The medium that was developed in section 2.8 (consisted of an ultra-pure water based solution containing 10% salt solution 17 (v/v) (Table 5), 1.5% MEM non-essential amino acids stock solution (v/v) (Table 3), 434.44 mg/L L-alanyl-L-glutamine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality)) supplemented with 0 mg/L, 10 mg/L, 50 mg/L, or 250 mg/L L-cysteine (Sigma-Aldrich, C7352) was used for preparing testing media. The cell number, monolayer formation, percentage of mitotic cells, and viability of cells growing out of the *Litopenaeus Vannamei* shrimp lymphoid organ explants were analyzed at 0, 7, and 14 days of culture.

### 2.10. L-tyrosine supplementation

Tyrosine is a polar, non-essential amino acid incorporated into reactions such as phosphorylation/dephosphorylation important for cell signaling. The effect of L-tyrosine in cell culture medium on crustacean cell culture was evaluated. The new medium as described in section 2.9 (consisted of an ultra-pure water based solution containing 10% salt solution 17 (v/v) (Table 5), 1.5% MEM non-essential amino acids stock solution (v/v) (Table 3), 434.44 mg/L L-alanyl-L-glutamine (w/v), 30 mg/L L-cysteine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality)) supplemented with 0 mg/L, 10 mg/L, 50 mg/L, or 250 mg/L L-cysteine (Sigma-Aldrich, C7352) was used for preparing testing media. A series of measurements were taken at 0, 7, and 14 days of culture to determine the number of cells, the formation of monolayers, the percentage of mitotic cells, and the viability of the cells growing from *Litopenaeus Vannamei* shrimp lymphoid organ explants.

### 2.11. Biotin supplementation

Biotin is an essential vitamin that is important for amino acids and energy metabolism, fatty acid synthesis, regulation of transcription and DNA repair. A study was conducted to evaluate the effect of biotin in cell culture medium on crustacean cell culture. The medium that was developed in section 2.10 (consisted of an ultra-pure water based solution containing 10% salt solution 17 (v/v) (Table 5), 1.5% MEM non-essential amino acids stock solution (v/v) (Table 3), 434.44 mg/L L-alanyl-L-glutamine (w/v), 30 mg/L L-cysteine (w/v), 35 mg/L L-tyrosine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality)) supplemented with 0 mg/L, 0.02 mg/L, 0.2 mg/L, or 2 mg/L biotin (Sigma-Aldrich, B4639) was used for preparing testing media. The number of cells, the formation of monolayers, the percentage of mitotic cells, and the viability of the cells growing out of the *Litopenaeus Vannamei* shrimp lymphoid explants was examined after 0, 7, and 14 days of culture.3. **Development of cell cultures**

The shrimp were disinfected by immersion in 4.0% hypochlorite solution and 70% ethanol prepared in cold seawater (3.5% salinity) for 2 min, respectively. Finally, the animals were rinsed several times in sterile cold seawater. The shrimp's lymphoid organ consists of two white small ovoid-shaped tissues between the lateral side of the stomach and the anterior edge of the hepatopancreas. The shrimp ovary is a bilaterally symmetrical organ extending in the mature animal for nearly its entire length, usually from the cardiac region of the stomach to the telson. The shrimp embryos were provided by collecting released fertilized eggs after artificial insemination of female shrimp (Lai et al., 2005). The organs were rinsed in an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 2000 mg/L sodium bicarbonate (w/v), 1.5 g/L glucose (w/v), 1.5 g/L lactalbumin (w/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) four times and chopped into 0.5 mm³ cubic explant. All the explants were transferred into wells of 24-well plates with 500 µl of culture medium per well and after 12 h post seeding another 500 µl of culture medium consisted of an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 5% ultrafiltered shrimp hemolymph, 2000 mg/L sodium bicarbonate (w/v), 1.5 g/L glucose (w/v), 1.5 g/L lactalbumin (w/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) added into each well. The cultures were incubated at 27 °C and examined every day with an inverted microscope. One third of the culture medium was changed every 3 days.

The lobsters were disinfected by immersion in 4.0% hypochlorite solution and 70% ethanol prepared in cold seawater (3.5% salinity) for 2 min, respectively. Finally, the animals were rinsed several times in sterile cold seawater. The hematopoietic tissue of the lobster is a thin layer of tissue loosely bound to the dorsal surface of the foregut.

The organ was rinsed in an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 2000 mg/L sodium bicarbonate (w/v), 1.5 g/L glucose (w/v), 1.5 g/L lactalbumin (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) four times and chopped into 0.5 mm₃ cubic explant. All the explants were transferred into wells of 24-well plates with 500 µl of culture medium per well and after 12 h post seeding another 500 µl of culture medium consisted of an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 5% ultrafiltered shrimp hemolymph, 2000 mg/L sodium bicarbonate (w/v), 1.5 g/L glucose (w/v), 1.5 g/L lactalbumin (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) added into each well. The cultures were incubated at 27 °C and examined every day with an inverted microscope. One third of the culture medium was changed every 3 days.

### 4. Monolayer formation, cell viability, and cell counting

Monolayer formation measurement was done by measuring the cell covered area using Digimizer Image Analysis Software by analyzing pictures taken at different time points of cell cultures. Hoechst and EMA staining were performed to count total and dead cells. The cultures on glass inserts were subsequently (i) incubated with 200 µl EMA solution (20 µg/ml in a shrimp specific medium (Table 7), Invitrogen) for 30 min in dark and on ice, (ii) exposed to candescent light for 10 min on ice, (iii) fixed with 500 µl of 4% paraformaldehyde for 10 min, (iv) incubated with 200 µl Hoechst solution (10 µg/ml in PBS, Sigma Aldrich) for 10 min, (v) washed once with crustacean PBS (371.9±25 mM NaCl, 8 mM Na₂HPO₄, 2 mM KH₂PO₄, and 2.7 mM KCl, PH: 7.2-7.4) and once with UP and finally mounted upside down on top of 2 µl of glycerin-DABCO on slides. All samples were analyzed using a fluorescent microscope (x40 objective) (Leica Microsystems DMRBE Wetzlar, Germany).

### 5. Subculture

Crustacean cells were cultured on 0.5% gelatin coated plates. 0.5% (w/v) gelatin solution was prepared by dissolving gelatin in tissue culture grade ultra-pure water. The solution was sterilized by autoclaving at 121 °C, 15 psi for 30 minutes. Culture surface was coated with 5-10 µL gelatin solution/cm² (i.e., 250-500 µg gelatin/cm²). The plates were allowed to dry at least 2 hours before introducing cells and medium. Explants were transferred into wells of gelatin coated 24-well plates with 500 µl of culture medium per well and after 12 h post seeding another 500 µl of shrimp cell culture medium was added into each well. The cultures were incubated at 27 °C. Half of the medium was changed every 3 days. To detach and subculture cells after one week post seeding from the surface, 1.5 mg/ml Collagenase IV plus 3 mM CaCl₂ dissolved in crustacean PBS (371.9±25 mM NaCl, 8 mM Na₂HPO₄, 2 mM KH₂PO₄, 2.7 mM KCI, with PH: 7.2-7.4) have been used. Collagenase IV is known as an enzyme which destroys the gelatin coating.

Crustacean PBS (CPBS) is a very efficient and harmless solution for shrimp cells. It contains 371.9 mM NaCl, 8 mM Na₂HPO₄, 2 mM KH₂PO₄, 2.7 mM KCl, with PH: 7.2-7.4. It can be used for washing cells and explants during preparing cell culture and subculture. The key elements are the osmolality (835±55 mmol/kg) and buffering system (to stabilize pH (Na₂HPO₄ and KH₂PO₄)) which are essential to make it work. Sodium chloride and potassium chloride are also important elements for a normal cell physiology. Crustacean PBS contains 371.9±25 mM NaCl, 8 mM Na₂HPO₄, 2 mM KH₂PO₄, 2.7 mM KCl, with PH: 7.2-7.4. Shrimp lymphoid organ cell cultures were sub-cultured every three days and cultured on gelatin coated plates.

### 6. Viral infection

### 6.1. WSSV inoculum preparation

50 g shrimp body without stomach and hepatopancreas from moribund WSSV (Thailand strain) infected shrimps was minced in 100 ml shrimp PBS on ice. The extract was centrifuged at 5500 g for 30 min at 4 °C. Then, supernatant was collected and passed through a 0.45 µm membrane (Sarstedt). The suspension was aliquoted in 1.5 ml sterile tubes and stored at -70°C.This WSSV stock (10⁸ SID50/ml) was diluted 10 times in a medium consisted of an ultra-pure water based solution containing 10% salt solution 17 (v/v) (Table 5), 1.5% MEM non-essential amino acids stock solution (v/v) (Table 3), 2% MEM vitamin solution (100X) (v/v) (Table 4), 5% ultrafiltered shrimp hemolymph, 2000 mg/L sodium bicarbonate (w/v), 1.5 g/L glucose (w/v), 1.5 g/L lactalbumin (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality), before use. The negative stock was prepared from healthy shrimp following the same procedure.

### 6.2. WSSV detection

WSSV infected cells were revealed in lymphoid cell cultures by indirect immunofluorescence (IIF). The lymphoid cell cultures (15 explants/well (1.9 cm²)) were prepared on gelatin (0.5%) coated glass inserts in 24-well plates. At 14 days of culture, 200 µl of WSSV solution (10^{6.3} SID50) was added to each well with lymphoid cell culture and incubated at 27 °C for 1 h. At the same time, the negative control was inoculated with 200 µl of healthy shrimp solution per well. Then, the inoculum was removed, and cells were washed three times with medium. One ml of medium was added to each well and the plates were incubated at 27 °C. Cell cultures were fixed at 0 h, 24 h and 48 h post inoculation and stained. In brief, the samples were fixed in 200 µl of 4% paraformaldehyde for 10 min and then in 200 µl of 0.1% Triton X-100 for 5 min at room temperature. After one washing with PBS for 5 min, the cell cultures were incubated in 200 µl of monoclonal antibody w29 (1:100 in PBS, Chaivisuthangkura et al., 2004) which is directed against WSSV viral protein VP28 at 37 °C for 1 h. Then, after three washes with PBS for 5 min, they were incubated in goat anti-mouse IgG-FITC (1:100 in PBS, Sigma Aldrich) at 37 °C for 1 h. After three washes with PBS for 5 min, the cell cultures were incubated in Hoechst solution (10 µg/ml in PBS, Sigma Aldrich) for 10 min. After three washings with PBS each time 5 minutes and one washing with UP water for 5 seconds, the cultures were mounted on slides with glycerin-DABCO and stored at 4 °C. The inoculated cell cultures were analyzed with a confocal fluorescence microscope (Leica TCS SP2 Laser scanning spectral confocal system, Leica microsystems GmbH, Germany). All images were processed and analysed by ImageJ software.

### 7. Statistical analysis

All results given in this paper were average values from three independent replicates with standard deviation. The effects of different treatments were statistically analyzed by ANOVA in SPSS 26.0. Differences were considered significant at *P < 0.05.*

### Results

### 1. Medium optimization

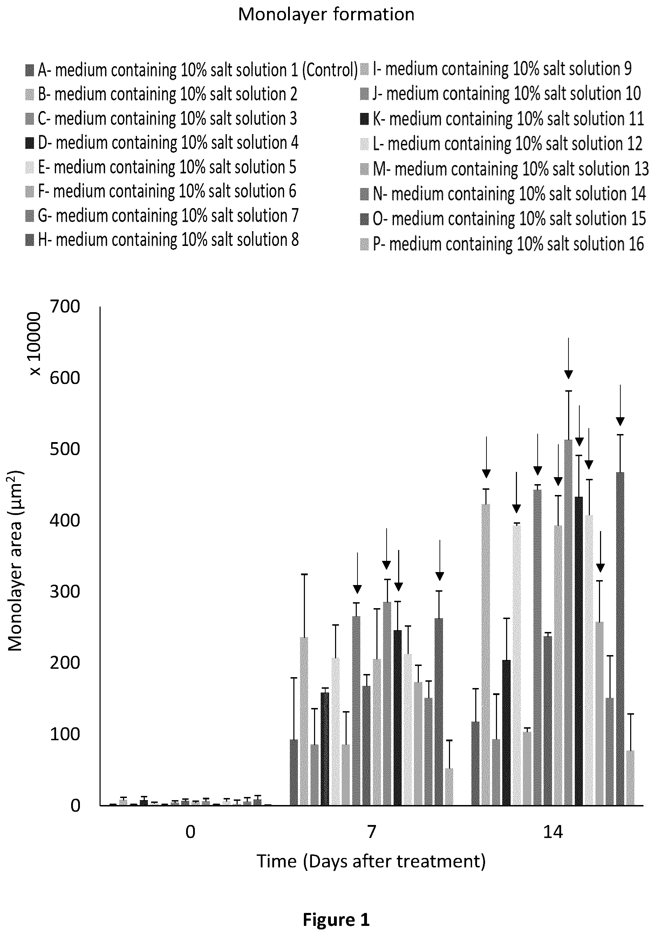

### 1.1. Inorganic salts and osmolality adjustment (Figure 1)

In this experiment, the effect of different inorganic salt solutions (Table 2) on the monolayer formation of the outgrowing cells of the shrimp lymphoid organ explants was examined. Salt solution 1 known as Chen's salt has been used before by several researcher for culturing crustacean cells (Li et al., 2014), so the 2X L15 medium containing the salt solution 1 has been used as reference. The osmolality of 2X L15 with 10% salt solution 1 was 927 mmol/kg while the osmolality of 2X L15 medium with 10% salt solutions 2 to 12 was 835±55 mmol/kg which is the same as *Litopenaeus Vannamei* shrimp hemolymph. Overall, cells growing with salt solution 10 showed better results on the monolayer formation compared to the other salt solutions. A significant difference was obtained at 14 days post seeding the explants in terms of monolayer formation of cells growing out of the shrimp lymphoid organ explant *(p-value* < 0.05) in media containing salt solutions 2, 5, 7, 9, 10, 11, 12, 13 or 15 compared to the reference (salt solution 1). The salt solution 10 gave the best results and was selected for the following experiments.

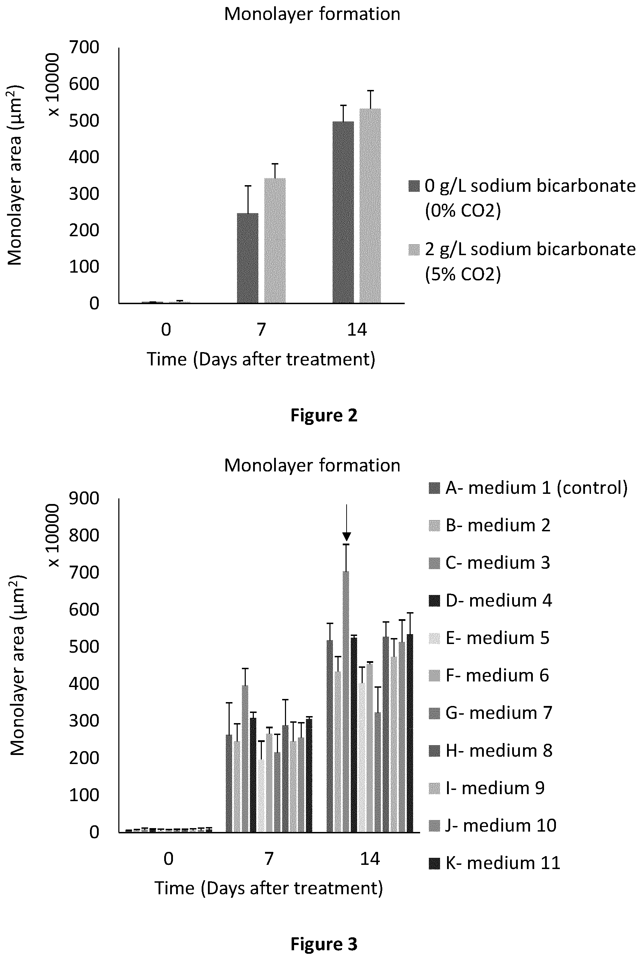

### 1.2. Buffering system adjustment (Figure 2)

In this experiment, the effect of 2 g/L sodium bicarbonate in the 2xL15 medium and 5% CO₂ in the air on the formation of a monolayer by cells growing out of the shrimp lymphoid organ explant was evaluated. Cells cultured in 2X L15 medium containing 2 g/L sodium bicarbonate at 5% CO₂ gave better results compared to cells cultured without sodium bicarbonate and in ambient air. Although the difference between reference and treatment groups was not statically significant *(p-value < 0.05),* medium was supplemented with sodium bicarbonate in the following experiments.

### 1.3. Amino acids adjustment

In this study we examined the effect of media containing different amino acid solutions (Table 3) on the monolayer formation of cells at 0, 7, and 14 days of culture. The results showed a significant difference in monolayer formation on day 14 of culture when medium containing MEM non-essential amino acids solution was used compared to the other solutions (*p-value < 0.05*) (Figure 3).

The medium containing MEM non-essential amino acids (Medium 3, Table 6) had the best performance when used to culture shrimp lymphoid organ cell culture. In order to obtain the optimal concentration of MEM non-essential amino acids in medium, the effect of five different media containing 0%, 0.5%, 1.5%, 5%, and 10% of MEM non-essential amino acids stock solution (Table 3) was evaluated.

The composition of all the media were the same except in terms of the amino acids (Table 8). Media 2, 3, 4, 8, 9, 10, and 11 were prepared by making an ultra-pure water based solution containing different concentrations of amino acids (Table 8), 10% salt solution 17 (v/v) (Table 5), 1.57% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality). Media 1, 5, 6, and 7 were prepared by supplementing 2X L15 medium with amino acids (Table 6), in addition to 10% salt solution 10 (v/v) (Table 5), 10% fetal calf serum (v/v), 2000 mg/L sodium bicarbonate (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

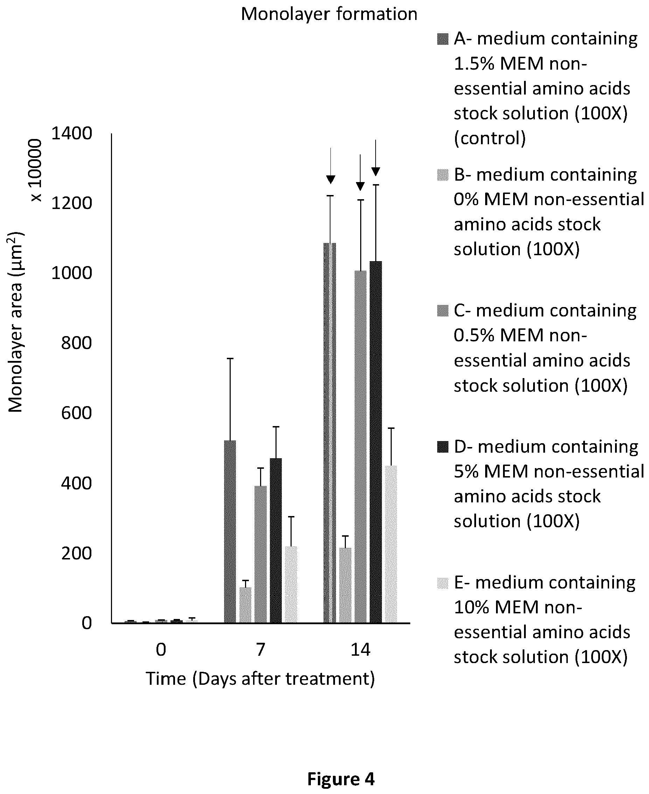

Shrimp lymphoid organ explants were cultured in the different media and incubated at 27°C, 5% CO₂ (pH 7.2-7.4). The formation of a monolayer of cells growing out of the shrimp lymphoid organ explants were analyzed at 0, 7, and 14 days of culture. The results showed a significant difference in monolayer formation on day 14 of culture when media containing 0.5%, 1.5%, or 5% MEM non-essential amino acids solution (Table 8) were used compared to the other solutions *(p-value < 0.05*) (Figure 4).

**Table 8. Different media containing various concentrations of MEM non-essential amino acids stock solution used for culturing cells growing out of shrimp lymphoid organ explants.**

| **Amino acids** | **Concentration (mg/L) of amino acids in media containing different percentages of MEM non-essential amino acids stock solution (100X)** | | | | |
|---|---|---|---|---|---|
| | **1.5% (reference)** | **0%** | **0.5%** | **5%** | **10%** |
| Glycine | 11.25 | 0 | 3.75 | 37.5 | 75 |
| alanine | 13.35 | 0 | 4.45 | 44.5 | 89 |
| proline | 17.25 | 0 | 5.75 | 57.5 | 115 |
| serine | 15.75 | 0 | 5.25 | 52.5 | 105 |
| asparagine | 19.8 | 0 | 6.6 | 66 | 132 |
| aspartic acid | 19.95 | 0 | 6.65 | 66.5 | 133 |
| glutamic acid | 22.05 | 0 | 7.35 | 73.5 | 147 |

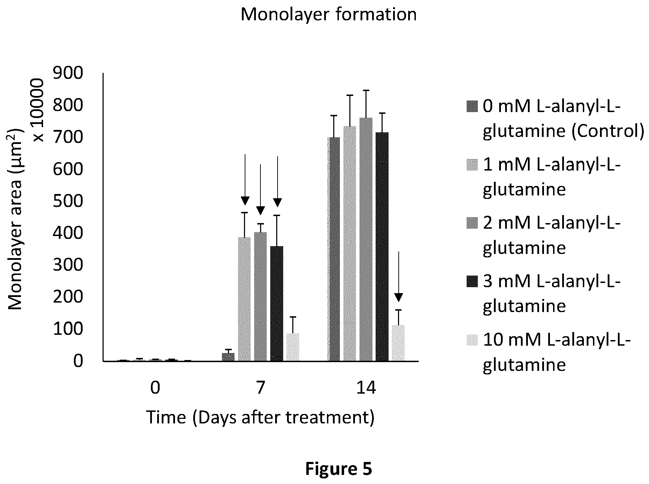

### 1.4. L-alanyl-L-glutamine adjustment (Figure 5)

In this experiment, the effect of medium consisting of ultra-pure water containing 10% salt solution 17 (v/v) (Table 5), 1.5% MEM non-essential amino acids stock solution (v/v) (Table 3), 1.57% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), 0.0025% amphotericin B (v/v) (0.25 mg/L amphotericin B) (pH 7.2-7.4 and 835±55 mmol/kg osmolality), and 0 mM, 1 mM, 2 mM, 3 mM, or 10 mM L-alanyl-L-glutamine was examined on the monolayer formation of cells growing out of the shrimp lymphoid organ explants. The highest concentration of L-alanyl-L-glutamine (10mM) was significantly toxic for the cells (*p-value* < *0.05*). The media containing 1 mM, 2 mM and 3 mM L-alanyl-L-glutamine were making larger monolayers at 7 days of culture compared to the reference medium containing 0 mM L-alanyl-L-glutamine. At 14 days of culture this difference disappeared. Medium containing 2 mM L-alanyl-L-glutamine was used in the following experiments.

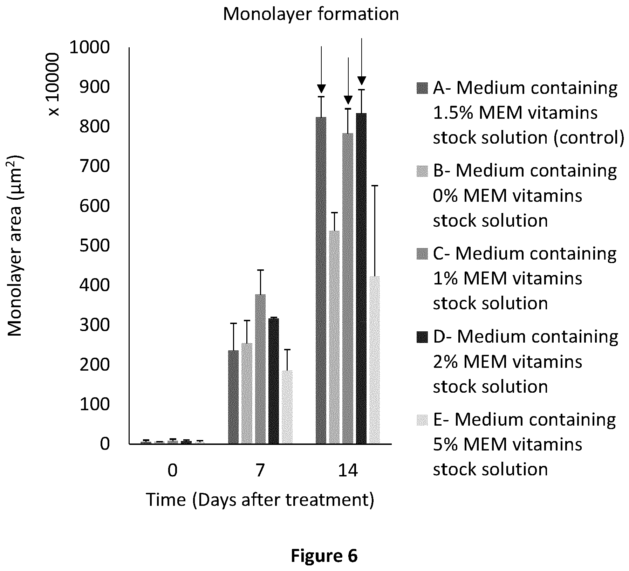

### 1.5. Vitamins' adjustment (Figure 6)

In this study we examined the effect of medium consisting of ultra-pure water, containing 10% salt solution 17 (v/v) (Table 5), 1.5% MEM non-essential amino acids stock solution (v/v) (Table 3), 10% fetal calf serum (v/v), 2000 mg/L sodium bicarbonate (w/v), 1413 mg/L galactose (w/v), 863.5 mg/L sodium pyruvate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) containing different concentration of MEM vitamin stock solution (100X) on the monolayer formation of cells growing out of explants at 0, 7, and 14 days of culture.

The results showed a significant difference in improvement of monolayer formation in day 14 of culture when media contain 1%, 1.5%, or 2% of the vitamin mixture compared to 0% or 5% (*p-value < 0.05*). Although the difference between media 1%, 1.5%, and 2% at day 14 post explant seeding was not significant, the results were the best with the 2% vitamin mixture, this vitamin mixture was chosen in the following experiments.

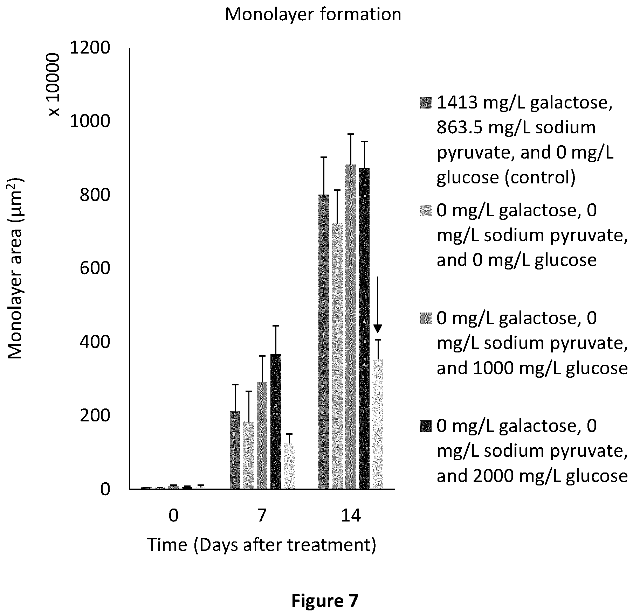

### 1.6. Glucose supplementation (Figure 7)

In this study, the effect of the medium (developed in the section "vitamins' adjustment") consisting of ultra-pure water, containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), 0.0025% amphotericin B (v/v) (0.25 mg/L amphotericin B) (pH 7.2-7.4 and 835±55 mmol/kg osmolality), and 0 g/L, 1 g/L, 2 g/L, or 10 g/L glucose was examined and compared with a reference medium consisting of the ultra-pure water containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 1413 mg/L galactose (w/v), 863.5 mg/L sodium pyruvate (w/v) (Table 6), 15.7 mg/L phenol red (w/v), 1% penicillin/streptomycin (v/v) (100000U/L penicillin,100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality).

Results showed that the highest concentration of glucose (10 g/L) is toxic for cells. The difference between media containing 0 g/L, 1 g/L, and 2 g/L glucose was not significant (*p-value < 0.05*) at 7 and 14 days of culture. However, as the 1-2 g/L was giving larger monolayers at 7 days of culture compared with the 0g/L, we used this concentration in the following experiments.

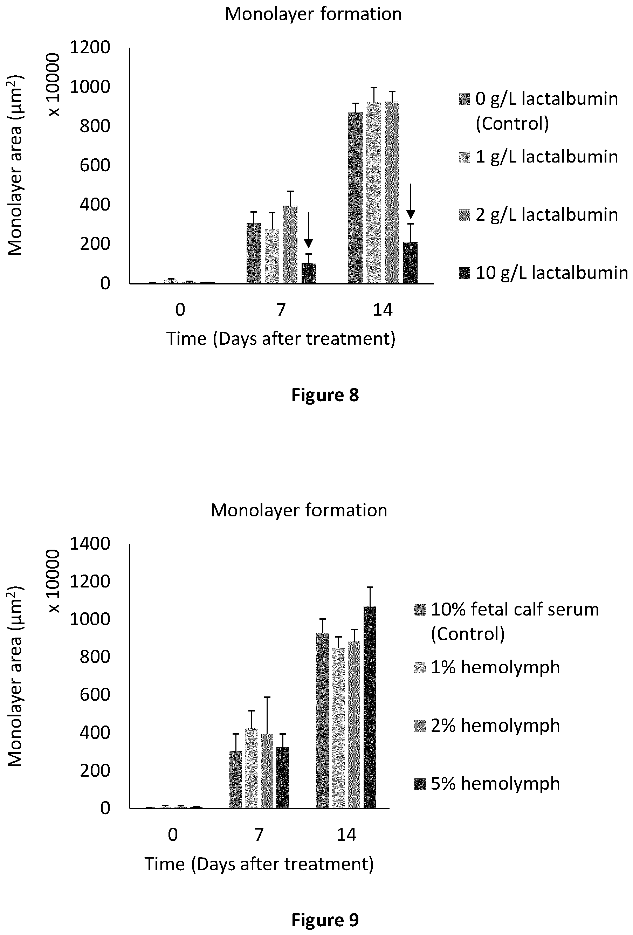

### 1.7. Lactalbumin supplementation (Figure 8)

In this experiment, the effect of medium (developed in the section "glucose supplementation") consisting of ultra-pure water containing 10% salt solution 17 (v/v) (Table 5), 1.5% MEM non-essential amino acids stock solution (v/v) (Table 3), 2% MEM vitamin solution (100X) (v/v) (Table 4), 10% fetal calf serum (v/v), 2000 mg/L sodium bicarbonate (w/v), 1.5 g/L glucose (w/v), 15.7 mg/L phenol red (w/v), 1% penicillin/streptomycin (v/v) (100000U/L penicillin,100 mg/L streptomycin), 0.5% gentamicin (v/v) (50 mg/L gentamicin), 0.0025% amphotericin B (v/v) (0.25 mg/L amphotericin B) (pH 7.2-7.4 and 835±55 mmol/kg osmolality), and 0 g/L, 1 g/L, 2 g/L, and 10 g/L lactalbumin was determined on the monolayer formation of cells growing out of the shrimp lymphoid organ explants. The highest concentration of lactalbumin (10 g/L) was toxic for the cells. The difference between media containing 0 g/L, 1 g/L, and 2 g/L lactalbumin was not significant at 7 and 14 days of culture (*p-value < 0.05).* As medium containing 2 g/L lactalbumin was better at 7 days of culture, this concentration was selected in the next experiments.

### 1.8. Hemolymph supplementation (Figure 9)

In this experiment, the effect of 10% fetal calf serum or 1%, 2%, or 5% shrimp hemolymph in the medium developed in the section "lactalbumin supplementation" consisting of ultra-pure water containing 10% salt solution 17 (v/v) (Table 5), 1.5% MEM non-essential amino acids stock solution (v/v) (Table 3), 2% MEM vitamin solution (100X) (v/v) (Table 4), 2000 mg/L sodium bicarbonate (w/v), 1.5 g/Lglucose (w/v), 1.5 g/L lactalbumin (w/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) on the monolayer formation of shrimp cells growing out of the shrimp lymphoid organ explants was determined. The difference between medium containing 10% fetal calf serum, 1% hemolymph, 2% hemolymph, and 5% hemolymph was not significant (*p-value < 0.05*). As the best results were obtained with 5% hemolymph, this medium was selected for the following experiments.

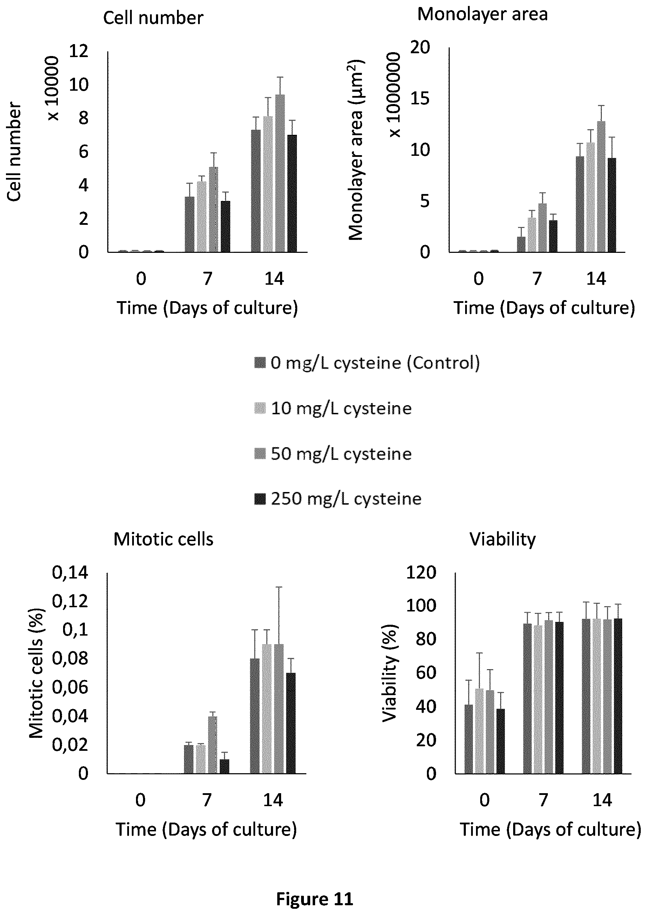

### 1.9. L-cysteine supplementation (Figure 11)

In this experiment, the effect of 0 mg/L, 10 mg/L, 50 mg/L, and 250 mg/L L-cysteine in the medium developed in the section "hemolymph supplementation" consisting of ultra-pure water containing 10% salt solution 17 (v/v) (Table 5), 1.5% MEM non-essential amino acids stock solution (v/v) (Table 3), 434.44 mg/L L-alanyl-L-glutamine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) was determined on the cell number, monolayer formation, percentage of mitotic cells, and viability of shrimp cells growing out of the shrimp lymphoid organ explants. The difference between medium containing 0 mg/L, 10 mg/L, 50 mg/L, and 250 mg/L L-cysteine was not significant (*p-value < 0.05).* As the medium containing 50 mg/L L-cysteine increased cells number, monolayer formation and percentage of mitotic cells, this medium was selected for the following experiments.

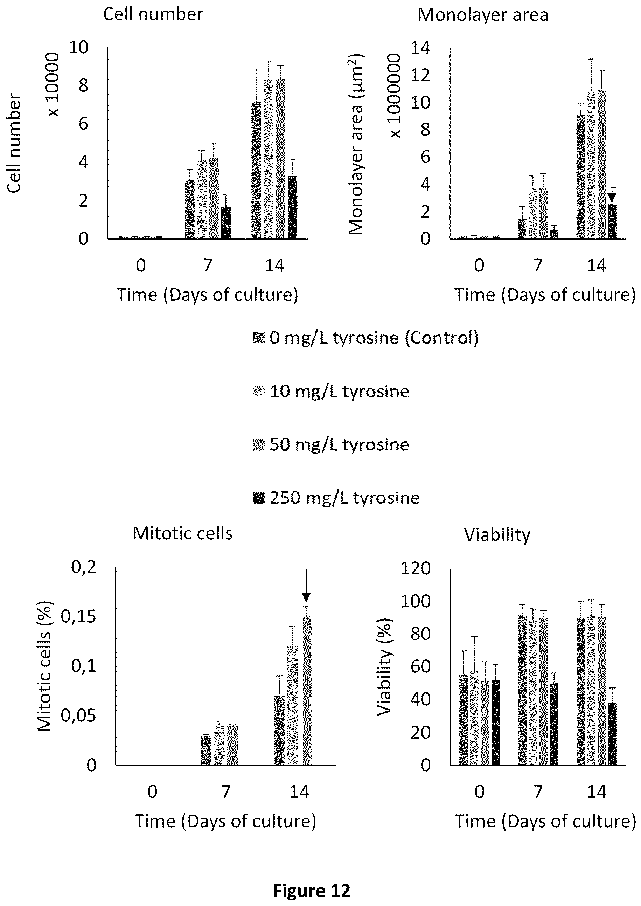

### 1.10. L-tyrosine supplementation (Figure 12)

This experiment was examined the effect of 0 mg/L, 10 mg/L, 50 mg/L, and 250 mg/L L-tyrosine in the medium developed in the section "L-cysteine supplementation" consisting of ultra-pure water containing 10% salt solution 17 (v/v) (Table 5), 1.5% MEM non-essential amino acids stock solution (v/v) (Table 3), 434.44 mg/L L-alanyl-L-glutamine (w/v), 30 mg/L L-cysteine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) on the cell number, monolayer formation, percentage of mitotic cells, and viability of shrimp cells growing out of the shrimp lymphoid organ explants. The difference between medium containing 0 mg/L, 10 mg/L, 50 mg/L, and 250 mg/L L-tyrosine was significant (*p-value < 0.05*)*.* As the medium containing 50 mg/L L-tyrosine significantly increased the percentage of mitotic cells, this medium was selected for the following experiments.

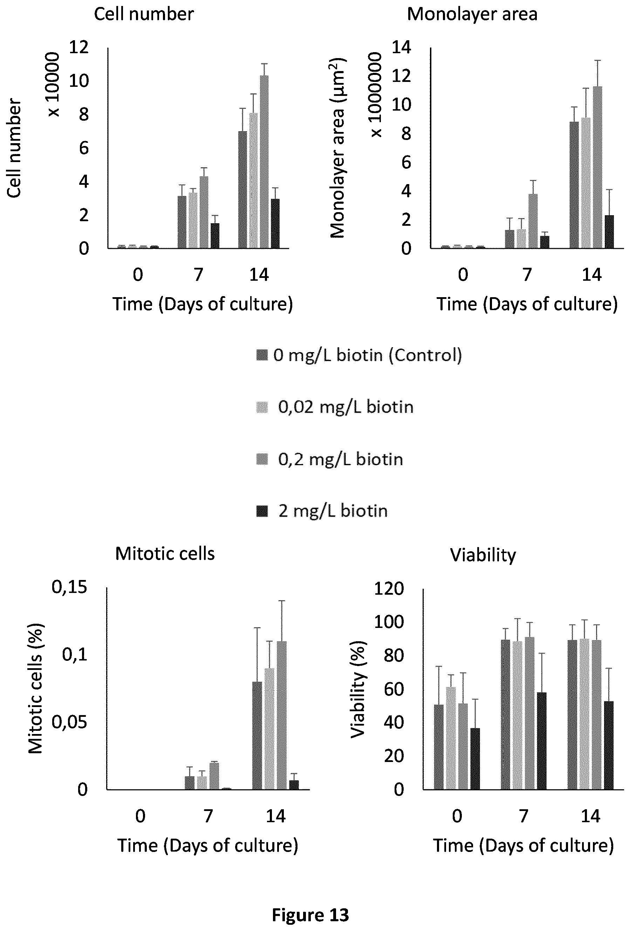

### 1.11. Biotin supplementation (Figure 13)

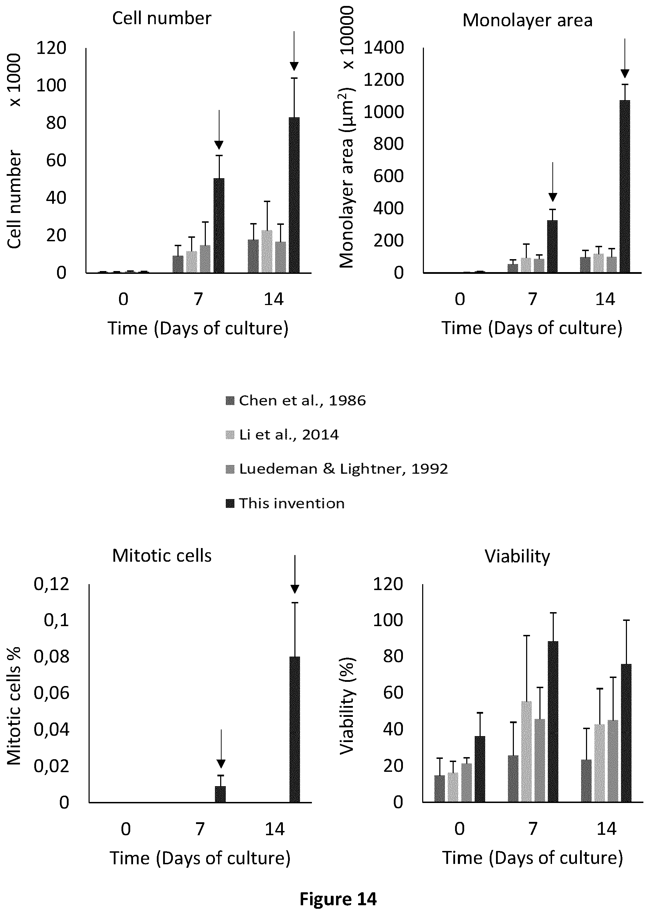

During this experiment, the effect of 0 mg/L, 0.02 mg/L, 0.2 mg/L, and 2 mg/L biotin in the medium developed in the section "L-tyrosine supplementation" consisting of ultra-pure water containing 10% salt solution 17 (v/v) (Table 5), 1.5% MEM non-essential amino acids stock solution (v/v) (Table 3), 434.44 mg/L L-alanyl-L-glutamine (w/v), 30 mg/L L-cysteine (w/v), 35 mg/L L-tyrosine (w/v), 2% MEM vitamin solution (100X) (v/v) (Table 4), 2000 mg/L sodium bicarbonate (w/v), 1.5 g/L glucose (w/v), 1.5 g/L lactalbumin (w/v), 5% hemolymph (v/v), 15.7 mg/L phenol red (w/v), 1% penicillin/streptomycin (v/v) (100000U/L penicillin, 100 mg/L streptomycin), 0.5% gentamicin (v/v) (50 mg/L gentamicin), and 0.0025% amphotericin B (v/v) (0.25 mg/L amphotericin B) (pH 7.2-7.4 and 835±55 mmol/kg osmolality) was evaluated on the cell number, monolayer formation, percentage of mitotic cells, and viability of shrimp cells growing out of the shrimp lymphoid organ explants. The difference between medium containing 0, 0.02, 0.2 and 2 mg/L biotin was not significant (*p-value* < *0.05*). As the medium containing 0.2 mg/L biotin increased cells number, monolayer formation and percentage of mitotic cells, this medium was selected for the following experiments.After optimization of the medium in terms of inorganic salts, osmolality, amino acids, vitamins, glucose, lactalbumin, and shrimp hemolymph an optimized medium was obtained which support a better monolayer formation and viability of cells growing out of the crustacean explants compared to other available media which are normally used for crustacean cell culture. In Table 9, the final composition of medium is available. This invention has been compared with three other research which have been done by other research groups to prove the efficiency of medium when used to culture shrimp lymphoid organ explants (Figure 14).

**Table 9. Final culture medium composition**

| **Components** | | **Optimum** | **Minimum** | **Maximum** |
|---|---|---|---|---|
| **Inorganic salts** | | | | |
| | NaCl | 8.27 g/L | 7.47 g/L | 9.07 g/L |
| | MgSO₄.7H₂O | 0.065 g/L | - | - |
| | MgCl₂.6H₂O | 0.064 g/L | - | - |
| | CaCl₂.2H₂O | 0.271 g/L | - | - |
| | KCl | 0.162 g/L | - | - |
| | KH₂PO₄ | 0.00942 g/L | - | - |
| | Na₂HPO₄ | 0.0298 g/L | - | - |

| **Amino acids** | | | | |
|---|---|---|---|---|
| | Glycine | 11.25 mg/L | 3.75 mg/L | 37.5 mg/L |
| | L-Alanine | 13.35 mg/L | 4.45 mg/L | 44.5 mg/L |
| | L-Asparagine | 19.8 mg/L | 6.6 mg/L | 66 mg/L |
| | L-Aspartic acid | 19.95 mg/L | 6.65 mg/L | 66.5 mg/L |
| | L-Glutamic Acid | 22.05 mg/L | 7.35 mg/L | 73.5 mg/L |
| | L-Proline | 17.25 mg/L | 5.75 mg/L | 57.5 mg/L |
| | L-Serine | 15.75 mg/L | 5.25 mg/L | 52.5 mg/L |
| | L-Cysteine | 30 mg/L | 10 mg/L | 50 mg/L |
| | L-Tyrosine | 30 mg/L | 10 mg/L | 50 mg/L |
| | L-alanyl-L-glutamine | 434.44 mg/L | 217.22 mg/L | 651.66 mg/L |
| | | | | |

| **Vitamins** | | | | |
|---|---|---|---|---|
| | Choline chloride | 1.57 mg/L | 1 mg/L | 2 mg/L |
| | D-Calcium pantothenate | 1.57 mg/L | 1 mg/L | 2 mg/L |
| | Folic Acid | 1.57 mg/L | 1 mg/L | 2 mg/L |
| | Nicotinamide | 1.57 mg/L | 1 mg/L | 2 mg/L |
| | Pyridoxal hydrochloride | 1.57 mg/L | 1 mg/L | 2 mg/L |
| | Riboflavin | 0.15 mg/L | 0.1 mg/L | 0.2 mg/L |
| | Thiamine hydrochloride | 1.57 mg/L | 1 mg/L | 2 mg/L |
| | i-Inositol | 3.14 mg/L | 2 mg/L | 4 mg/L |
| | Biotin | 0.11 mg/L | 0.02 mg/L | 0.2 mg/L |

| **Supplements** | | | | |
|---|---|---|---|---|
| | D-Glucose | 1.5 g/L | 1 g/L | 2 g/L |
| | Lactalbumin | 1.5 g/L | 1 g/L | 2 g/L |
| | Ultra-filtered crustacean hemolymph | 5% | - | - |

| **Antibiotics** | | | | |
|---|---|---|---|---|
| | Penicillin | 100000 U/L | - | - |
| | Streptomycin | 100 mg/L | - | - |
| | Gentamicin | 50 mg/L | - | - |
| | Amphotericin B | 0.25 mg/L | - | - |
| **Sodium bicarbonate** | | 2 g/L | - | - |
| **Phenol red** | | 15.7 mg/L | - | - |
| CO₂ | | 5% | - | - |
| **Osmolality** | | 835mmol/kg | 780 mmol/kg | 890 mmol/kg |
| **pH** | | 7.3 | 7.2 | 7.4 |

### 2. Origin of cells

Explants from various organs of different crustacea, including shrimp (*Litopenaeus Vannamei*) and lobster (*Homarus americanus*) have been used for the preparation of cell cultures. The selected medium was used for these cultures.

### 2.1. Shrimp lymphoid organ

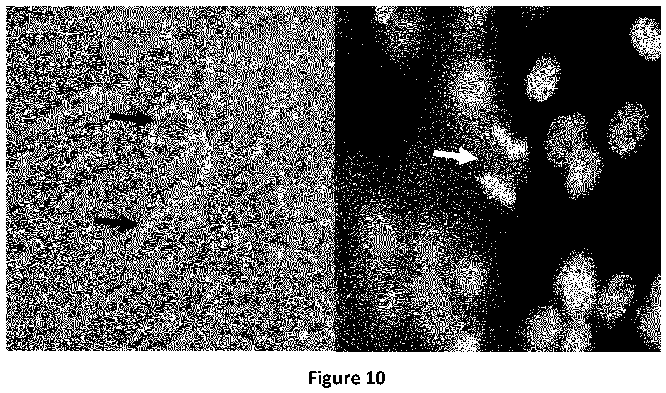

The newly defined medium (see Table 9) supported cells growing out of the shrimp lymphoid organ explant and allowed the cells to proliferate. The monolayer size and cell number increased over time and the viability stayed at 76% after 14 days of culture (Figures 10 and 14).

### 2.2. Shrimp ovary

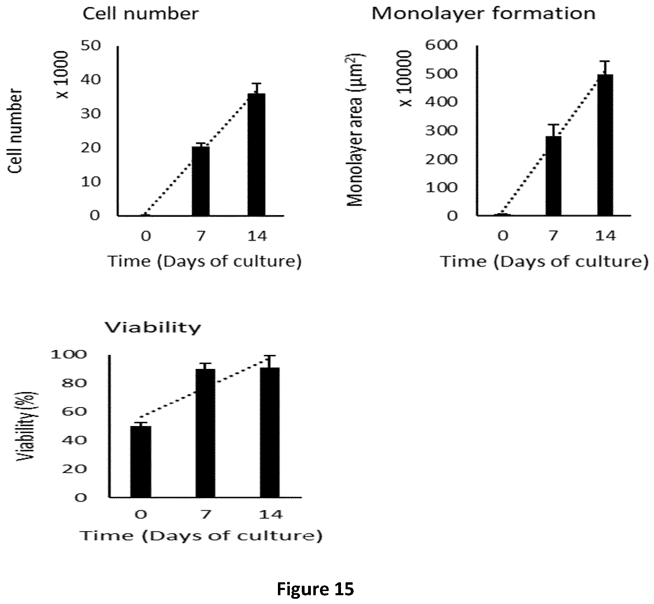

The newly defined medium supported cells growing out of the shrimp ovary organ explants and allowed the cells to proliferate. The monolayer size and cell number increased over time and the viability stayed at 91% after 14 days of culture (Figure 15).

### 2.3. Shrimp embryos

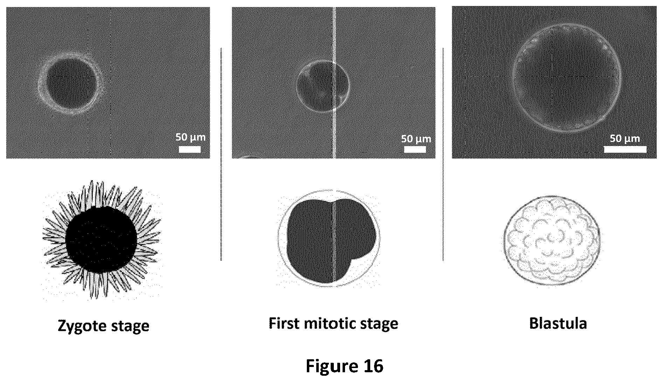

The results showed that the medium can support embryonic cells development through different embryonic stages (Figure 16).

### 2.4. Lobster hematopoietic organ

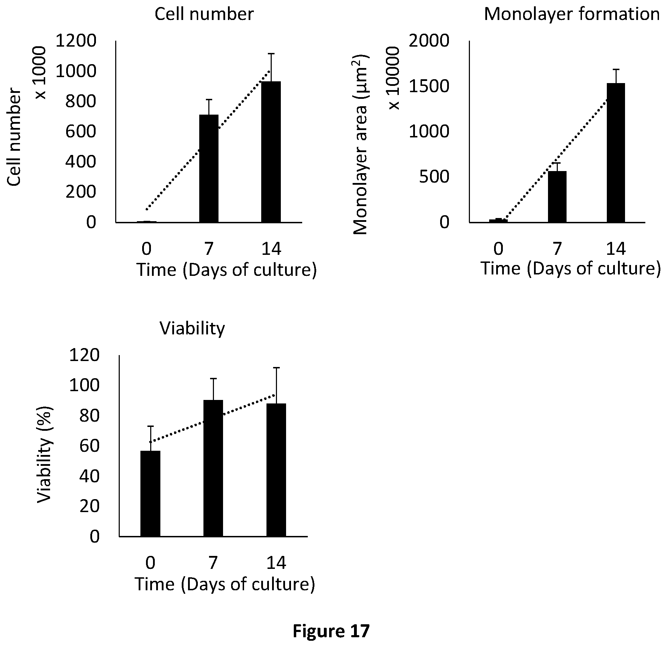

The newly defined medium supported cells growing out of the lobster hematopoietic organ explants. The monolayer size and cell number increased over time and the viability stayed at 88% after 14 days of culture (Figure 17).

### 3. Subculture

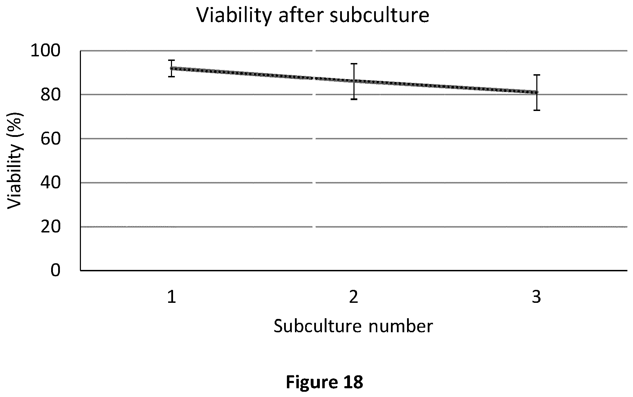

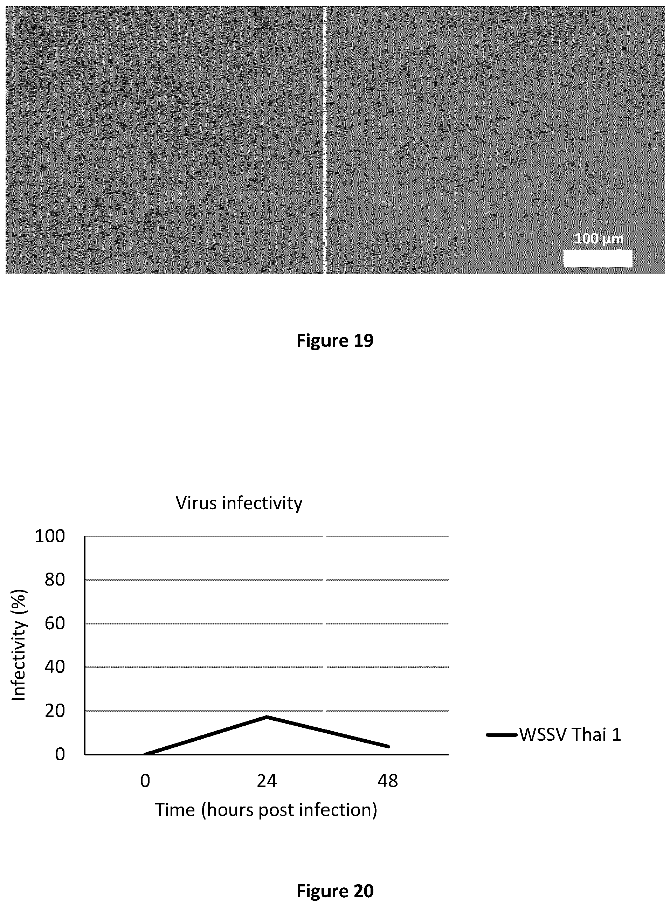

In this experiment, the effect of a subculture method by culturing crustacean cells on 0.5% gelatin coated plates and detaching cells using 1.5 mg/ml Collagenase IV plus 3 mM CaCl₂ dissolved in crustacean PBS was examined. The cells were subcultured every three days. The results showed that the cell viability remained above 80% after three subcultures (Figures 18 and 19).

### 4. Viral infection

Infecting 14 days' old shrimp cells migrated out of lymphoid organ explant using WSSV Thai 1 virus showed 17.2%, and 3.7% infectivity after 24 and 48 hours post infection with 200µL of 10^{6.3} SID50 of WSSV. The low infection ratio at 48 hours post infection was the result of the detachment of the dead infected cells (Figure 20).

### 5. Conclusion

The present invention discloses a crustacean-specific cell culture medium which supports proliferation of the crustacean cells in vitro in a superior manner. Indeed, the latter medium significantly increases monolayer formation, viability, and number of cells growing out of crustacea explants at 14 days of culture compared to other media. The medium has been optimized in terms of inorganic salts, osmolality, buffering system, amino acids, glutamine, vitamins, glucose, lactalbumin, and was supplemented with hemolymph. The present invention shows that inorganic salts, osmolality, amino acids, and vitamins optimization had a significant effect on improving the performance of culture medium on crustacean cell culture. Furthermore, a buffered solution for washing cells and explants during the preparation of the cell cultures and subcultures has been disclosed. Also, a detachment solution which can detach shrimp cells gently with above 80% viability after the first three subcultures has been disclosed. This detachment solution is adapted to shrimp cells and is safe and harmless for cells in culture. In addition, the cell cultures of the present invention can be used as a tool to perform research and diagnosis of crustacean pathogens.

### References

Achs, M., and D. Garfinkel. "Regulation and control in physiological systems." In International Federation of automatic control symposium, pp. 19-21. 1973.

Arora, M. (2013). Cell culture media: a review. Mater methods, 3(175), 24.

Aston, N. S., N. Watt, I. E. Morton, M. S. Tanner, and G. S. Evans. "Copper toxicity affects proliferation and viability of human hepatoma cells (HepG2 line)." Human & experimental toxicology 19, no. 6 (2000): 367-376.

Atanassov, Christo L., Nikolaus Seiler, and Gérard Rebel. "Reduction of ammonia formation in cell cultures by L-alanyl-L-glutamine requires optimization of the dipeptide concentration." Journal of biotechnology 62, no. 2 (1998): 159-162.

Atmaca, Gulizar. "Antioxidant effects of sulfur-containing amino acids." Yonsei medical journal 45, no. 5 (2004): 776-788.

Baltz, Jay M. "Media composition: salts and osmolality." Embryo Culture (2012): 61-80.

Barnes, David, and Gordon Sato. "Serum-free cell culture: a unifying approach." Cell 22, no. 3 (1980): 649-655.

Bell, Thomas A., and Donald V. Lightner. "A handbook of normal penaeid shrimp histology." (1988).

Bjare, Ulf. "Serum-free cell culture." Pharmacology & therapeutics 53, no. 3 (1992): 355-374.

Borle, André B. "Kinetic analysis of calcium movements in cell culture." The Journal of membrane biology 10, no. 1 (1972): 45-66.

Bram, Stanley, Patrick Froussard, Marcelle Guichard, Claude Jasmin, Yvette Augery, Françoise Sinoussi-Barre, and Wayne Wray. "Vitamin C preferential toxicity for malignant melanoma cells." Nature 284, no. 5757 (1980): 629-631.

Brody, Michel D., and Ernest S. Chang. "Development and utilization of crustacean long-term primary cell cultures: ecdysteroid effects in vitro." Invertebrate reproduction & development 16, no. 1-3 (1989): 141-147.

Burg, Maurice B., Eugene D. Kwon, and Dietmar KÜltz. "Regulation of gene expression by hypertonicity." Annual Review of Physiology 59, no. 1 (1997): 437-455.

Butler, M. (2004). Animal cell culture and technology. Taylor & Francis.

Carinhas, Nuno, Tiago M. Duarte, Laura C. Barreiro, Manuel JT Carrondo, Paula M. Alves, and Ana P. Teixeira. "Metabolic signatures of GS-CHO cell clones associated with butyrate treatment and culture phase transition." Biotechnology and bioengineering 110, no. 12 (2013): 3244-3257.

Carrillo-Cocom, L. M., T. Genel-Rey, D. Araiz-Hernandez, F. López-Pacheco, J. Lopez-Meza, M. R. Rocha-Pizana, A. Ramírez-Medrano, and M. M. Alvarez. "Amino acid consumption in naive and recombinant CHO cell cultures: producers of a monoclonal antibody." Cytotechnology 67, no. 5 (2015): 809-820.

Chaivisuthangkura, Parin, Jarasporn Tangkhabuanbutra, Siwaporn Longyant, Weerawan Sithigorngul, Sombat Rukpratanporn, Piamsak Menasveta, and Paisarn Sithigorngul. "Monoclonal antibodies against a truncated viral envelope protein (VP28) can detect white spot syndrome virus (WSSV) infections in shrimp." ScienceAsia 30 (2004): 359-363.

Chamberlain, G. W. "History of shrimp farming." The Shrimp Book Nottingham University Press, United Kingdom (2010): 1-34.

Chang, Ernest S., and Michael D. Brody. "Crustacean organ and cell culture." In Advances in cell culture, vol. 7, pp. 19-86. Elsevier, 1989.

Chen, Jiann-Chu, and Peng-Gek Chia. "Oxyhemocyanin, protein, osmolality and electrolyte levels in the hemolymph of Scylla serrata in relation to size and molt cycle." Journal of Experimental Marine Biology and Ecology 217, no. 1 (1997): 93-105.

Chen, Peifeng, and Sarah W. Harcum. "Effects of elevated ammonium on glycosylation gene expression in CHO cells." Metabolic Engineering 8, no. 2 (2006): 123-132.

Chen, S. N., and C. S. Wang. "Establishment of cell culture systems from penaeid shrimp and their susceptibility to white spot disease and yellow head viruses." Methods in Cell Science 21, no. 4 (1999): 199-206.

Chen, S. N., Shau-Chi CHI, G. H. Kou, and I-Chiu Liao. "Cell culture from tissues of grass prawn, Penaeus monodon." Fish pathology 21, no. 3 (1986): 161-166.

Clapham, David E. "Calcium signaling." Cell 80, no. 2 (1995): 259-268.

Claydon, Kerry. "Advances in crustacean cell culture." PhD diss., James Cook University, 2009.

Dakshinamurti, K. R. I. S. H. N. A. M. U. R. T. I., Lorraine Chalifour, and RAJINDER P. Bhullar. "Requirement for biotin and the function of biotin in cells in culture." Annals of the New York Academy of Sciences 447 (1985): 38-55.

Dakshinamurti, Krishnamurti. "Biotin-a regulator of gene expression." The Journal of nutritional biochemistry 16, no. 7 (2005): 419-423.

Dall, W. H. B. J., B. J. Hill, Peter C. Rothlisberg, and D. J. Sharples. "The biology of the Penaeidae." The biology of the Penaeidae. 27 (1990).

Dantas-Lima, J. J., Mathias Corteel, D. T. H. Oanh, Peter Bossier, Patrick Sorgeloos, and H. J. Nauwynck. "Development of two haemocyte culture systems (in attachment and in suspension) for shrimp immunity studies." Aquaculture 366 (2012): 17-26.

Duangsuwan, Pornsawan, Yotsawan Tinikul, Charoonroj Chotwiwatthanakun, Rapeepun Vanichviriyakit, and Prasert Sobhon. "Changes in the histological organization and spheroid formation in lymphoid organ of Penaeus monodon infected with yellow head virus." Fish & shellfish immunology 25, no. 5 (2008): 560-569.

Eagle, Harry. "Buffer combinations for mammalian cell culture." Science 174, no. 4008 (1971): 500-503.

Ellerton, H. David, Nerida F. Ellerton, and Heather A. Robinson. "Hemocyanin-a current perspective." Progress in Biophysics and Molecular Biology 41 (1983): 143-247.

Factor, Jan Robert, ed. Biology of the Lobster: Homarus americanus. Academic Press, 1995.

Fan, Ting-Jun, and Xiao-Feng Wang. "In vitro culture of embryonic cells from the shrimp, Penaeus chinensis." Journal of experimental marine biology and ecology 267, no. 2 (2002): 175-184.

Farber, John L. "The role of calcium in cell death." Life sciences 29, no. 13 (1981): 1289-1295.

Farber, John L. "The role of calcium ions in toxic cell injury." Environmental health perspectives 84 (1990): 107-111.

Fomina-Yadlin, Dina, John J. Gosink, Rebecca McCoy, Brian Follstad, Arvia Morris, Chris B. Russell, and Jeffrey T. McGrew. "Cellular responses to individual amino-acid depletion in antibody-expressing and parental CHO cell lines." Biotechnology and bioengineering 111, no. 5 (2014): 965-979.

Francis, Geoffrey L. "Albumin and mammalian cell culture: implications for biotechnology applications." Cytotechnology 62, no. 1 (2010): 1-16.

Frandsen, Aase, and Arne Schousboe. "Time and concentration dependency of the toxicity of excitatory amino acids on cerebral neurones in primary culture." Neurochemistry international 10, no. 4 (1987): 583-591.

Freshney, R. I., and John Masters. Animal cell culture. Oxford University Press, 2000.

Freshney, R. lan. "Culture of specific cell types." Culture of animal cells: a manual of basic technique (2005).

Gao, Song, Taijun Yin, Beibei Xu, Yong Ma, and Ming Hu. "Amino acid facilitates absorption of copper in the Caco-2 cell culture model." Life sciences 109, no. 1 (2014): 50-56.

George, Sunil K., and Arun K. Dhar. "An improved method of cell culture system from eye stalk, hepatopancreas, muscle, ovary, and hemocytes of Penaeus vannamei." In Vitro Cellular & Developmental Biology-Animal 46, no. 9 (2010): 801-810.

Grace, T. D. C., and H. W. Brozostowski. "Analysis of the amino acids and sugars in an insect cell culture medium during cell growth." Journal of Insect Physiology 12, no. 6 (1966): 625-633.

Gstraunthaler, Gerhard, Toni Lindl, and Jan van der Valk. "A plea to reduce or replace fetal bovine serum in cell culture media." Cytotechnology 65, no. 5 (2013): 791-793.

Gudjonsson, T., R. Villadsen, L. Rønnov-Jessen, and O. W. Petersen. "Immortalization protocols used in cell culture models of human breast morphogenesis." Cellular and Molecular Life Sciences CMLS 61, no. 19 (2004): 2523-2534.

Haab, Brian B. "Antibody arrays in cancer research." Molecular & cellular proteomics 4, no. 4 (2005): 377-383.

Han, Qian, Pengtao Li, Xiongbin Lu, Zijuan Guo, and Huarong Guo. "Improved primary cell culture and subculture of lymphoid organs of the greasyback shrimp Metapenaeus ensis." Aquaculture 410 (2013): 101-113.

Hassell, T., S. Gleave, and M. Butler. "Growth inhibition in animal cell culture." Applied biochemistry and biotechnology 30, no. 1 (1991): 29-41.

Helgason, Cheryl D., and Cindy L. Miller. Basic cell culture protocols. Totowa, NJ.: Humana Press, 2005.

Hernández-lbáñez, Naiara, Ignacio Sanjuán, Miguel Ángel Montiel, Christopher W. Foster, Craig E. Banks, and Jesús Iniesta. "I-Cysteine determination in embryo cell culture media using Co (II)-phthalocyanine modified disposable screen-printed electrodes." Journal of Electroanalytical Chemistry 780 (2016): 303-310.

Hoffmann, Else K., and Philip B. Dunham. "Membrane mechanisms and intracellular signalling in cell volume regulation." International review of cytology 161 (1995): 173-262.

Holley, Robert W. "Control of growth of mammalian cells in cell culture." Nature 258, no. 5535 (1975): 487-490.

Hsu, Ya-Li, Yin-Hsiu Yang, Yu-Chin Chen, Ming-Chen Tung, Jen-Leih Wu, Mark H. Engelking, and Joann C. Leong. "Development of an in vitro subculture system for the oka organ (lymphoid tissue) of Penaeus monodon." Aquaculture 136, no. 1-2 (1995): 43-55.

Huang, L., R. N. Vellanki, J. Sugihara, X. Gao, L. Jing, A. Gower, S. Soltanieh et al. "L-alanyl-L-glutamine improves lung performance during ex vivo lung perfusion: from cellular mechanism to porcine donor lungs." The Journal of Heart and Lung Transplantation 39, no. 4 (2020): S197-S198.

lkonomou, L., Y-J. Schneider, and S. N. Agathos. "Insect cell culture for industrial production of recombinant proteins." Applied microbiology and biotechnology 62, no. 1 (2003): 1-20.

Ishaque, Adiba, and Mohamed Al-Rubeai. "Role of vitamins in determining apoptosis and extent of suppression by bcl-2 during hybridoma cell culture." Apoptosis 7, no. 3 (2002): 231-239.

Itami, Toshiaki, Minoru Maeda, Masakazu Kondo, and Yukinori Takahashi. "Primary culture of lymphoid organ cells and haemocytes of kuruma shrimp, Penaeus japonicus." Methods in cell science 21, no. 4 (1999): 237-244.

Jiang, You-Sheng, Wen-Bin Zhan, Shi-Biao Wang, and Jing Xing. "Development of primary shrimp hemocyte cultures of Penaeus chinensis to study white spot syndrome virus (WSSV) infection." Aquaculture 253, no. 1-4 (2006): 114-119.

Jiravanichpaisal, Pikul, Bok Luel Lee, and Kenneth Söderhäll. "Cell-mediated immunity in arthropods: hematopoiesis, coagulation, melanization and opsonization." Immunobiology 211, no. 4 (2006): 213-236.

Johnson, Phyllis Truth. Histology of the blue crab, Callinectes sapidus: a model for the Decapoda. Greenwood, 1980.

Jose, Seena, A. Mohandas, Rosamma Philip, and IS Bright Singh. "Primary hemocyte culture of Penaeus monodon as an in vitro model for white spot syndrome virus titration, viral and immune related gene expression and cytotoxicity assays." Journal of invertebrate pathology 105, no. 3 (2010): 312-321.

Kang, Sohye, Johanna Mullen, Les P. Miranda, and Rohini Deshpande. "Utilization of tyrosine-and histidine-containing dipeptides to enhance productivity and culture viability." Biotechnology and bioengineering 109, no. 9 (2012): 2286-2294.

Kaplan, J. Gordin. "Membrane cation transport and the control of proliferation of mammalian cells." Annual review of physiology 40, no. 1 (1978): 19-41.

Kasornchandra, J., R. Khongpradit, U. Ekpanithanpong, and S. Boonyaratpalin. "Progress in the development of shrimp cell cultures in Thailand." Methods in cell science 21, no. 4 (1999): 231-235.

Kasornchandra, Jiraporn, S. Boonyaratpalin, and T. Itami. "Detection of white-spot syndrome in cultured penaeid shrimp in Asia: Microscopic observation and polymerase chain reaction." Aquaculture 164, no. 1-4 (1998): 243-251.

King, Joseph E. "A study of the reproductive organs of the common marine shrimp, Penaeus setiferus (Linnaeus)." The Biological Bulletin 94, no. 3 (1948): 244-262.

Krebs, H. A., and G. Weber. "Advances in Enzyme Regulation." V. 7 (1972): 397.

Kruse, Carla R., Mansher Singh, Stefan Targosinski, Indranil Sinha, Jens A. Sørensen, Elof Eriksson, and Kristo Nuutila. "The effect of pH on cell viability, cell migration, cell proliferation, wound closure, and wound reepithelialization: In vitro and in vivo study." Wound Repair and Regeneration 25, no. 2 (2017): 260-269.

Kuroishi, Toshinobu, Luisa Rios-Avila, Valerie Pestinger, Subhashinee SK Wijeratne, and Janos Zempleni. "Biotinylation is a natural, albeit rare, modification of human histones." Molecular genetics and metabolism 104, no. 4 (2011): 537-545.

Lai, Qiu-ming, Hai-shi Wang, and Kong-zhong Fu. "Artificial insemination of Litopenaeus vannamei." MARINE SCIENCES-QINGDAO-CHINESE EDITION- 29, no. 10 (2005): 19.

Lande, Marc B., Joanne M. Donovan, and Mark L. Zeidel. "The relationship between membrane fluidity and permeabilities to water, solutes, ammonia, and protons." Journal of General Physiology 106, no. 1 (1995): 67-84.

Lane, Michelle, and David K. Gardner. "Amino acids and vitamins prevent culture-induced metabolic perturbations and associated loss of viability of mouse blastocysts." Human reproduction (Oxford, England) 13, no. 4 (1998): 991-997.

Lee, Juliana TY, Yang Leng, King L. Chow, Fuzeng Ren, Xiang Ge, Kefeng Wang, and Xiong Lu. "Cell culture medium as an alternative to conventional simulated body fluid." Acta Biomaterialia 7, no. 6 (2011): 2615-2622.

Leong, Dawn Sow Zong, Brian Kah Hui Teo, Janice Gek Ling Tan, Hayati Kamari, Yuan Sheng Yang, Peiqing Zhang, and Say Kong Ng. "Application of maltose as energy source in protein-free CHO-K1 culture to improve the production of recombinant monoclonal antibody." Scientific reports 8, no. 1 (2018): 1-12.

Lepe-Zuniga, Jose Luis, J. S. Zigler Jr, and Igal Gery. "Toxicity of light-exposed Hepes media." Journal of immunological methods 103, no. 1 (1987): 145-145.

Li, Caiwen, and Jeffrey D. Shields. "Primary culture of hemocytes from the Caribbean spiny lobster, Panulirus argus, and their susceptibility to Panulirus argus Virus 1 (PaV1)." Journal of invertebrate pathology 94, no. 1 (2007): 48-55.

Li, Wenfeng, Mathias Corteel, João José Dantas-Lima, Khuong Van Thuong, Vo Van Tuan, Peter Bossier, Patrick Sorgeloos, and Hans Nauwynck. "Characterization of a primary cell culture from lymphoid organ of Litopenaeus vannamei and use for studies on WSSV replication." Aquaculture 433 (2014): 157-163.

Li, Y., G. L. Sattler, and H. C. Pitot. "The effect of amino acid composition of serum-free medium on DNA synthesis in primary hepatocyte cultures in the presence of epidermal growth factor." In Vitro Cellular & Developmental Biology-Animal 31, no. 11 (1995): 867.

Lorenzi, Mara, Enrico Cagliero, and Silva Toledo. "Glucose toxicity for human endothelial cells in culture: delayed replication, disturbed cell cycle, and accelerated death." Diabetes 34, no. 7 (1985): 621-627.

Luedeman, RenéA, and Donald V. Lightner. "Development of an in vitro primary cell culture system from the penaeid shrimp, Penaeus stylirostris and Penaeus vannamei." Aquaculture 101, no. 3-4 (1992): 205-211.

Ma, Jie, Lingbing Zeng, and Yuanan Lu. "Penaeid shrimp cell culture and its applications." Reviews in Aquaculture 9, no. 1 (2017): 88-98.

Maeda, Minoru, Hiroyuki Saitoh, Eiichi Mizuki, Toshiaki Itami, and Michio Ohba. "Replication of white spot syndrome virus in ovarian primary cultures from the kuruma shrimp, Marsupenaeus japonicus." Journal of virological methods 116, no. 1 (2004): 89-94.

Malorni, Walter, Roberto Rivabene, and Paola Matarrese. "The antioxidant N-acetyl-cysteine protects cultured epithelial cells from menadione-induced cytopathology." Chemico-biological interactions 96, no. 2 (1995): 113-123.

Maranga, Luis, Ana S. Coroadinha, and Manuel JT Carrondo. "Insect cell culture medium supplementation with fetal bovine serum and bovine serum albumin: effects on baculovirus adsorption and infection kinetics." Biotechnology progress 18, no. 4 (2002): 855-861.

Martin, Gary G., Jo Ellen Hose, Maryanne Choi, Ron Provost, Gregg Omori, Nancy McKrell, and Garrett Lam. "Organization of hematopoietic tissue in the intermolt lobster, Homarus americanus." Journal of morphology 216, no. 1 (1993): 65-78.

McKee, Turney J., and Svetlana V. Komarova. "Is it time to reinvent basic cell culture medium?." (2017): C624-C626.

Mendon 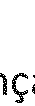 a, Ronaldo Zucatelli, Elizabeth Cristina de Oliveira, Carlos Augusto Pereira, and Ivo Lebrun. "Effect of bioactive peptides isolated from yeastolate, lactalbumin and NZCase in the insect cell growth." *Bioprocess and biosystems engineering* 30, no. 3 (2007): 157-164.

Messmer, Trudy O., and Delano V. Young. "The effects of biotin and fatty acids on SV3T3 cell growth in the presence of normal calf serum." Journal of Cellular Physiology 90, no. 2 (1977): 265-270.

Monteiro, Sandra Mariza, Nuno MS dos Santos, Margarida Calejo, António Fontainhas-Fernandes, and Mário Sousa. "Copper toxicity in gills of the teleost fish, Oreochromis niloticus: effects in apoptosis induction and cell proliferation." Aquatic toxicology 94, no. 3 (2009): 219-228.

Morth, J. Preben, Bjørn P. Pedersen, Morten J. Buch-Pedersen, Jens Peter Andersen, Bente Vilsen, Michael G. Palmgren, and Poul Nissen. "A structural overview of the plasma membrane Na+, K+-ATPase and H+-ATPase ion pumps." Nature reviews Molecular cell biology 12, no. 1 (2011): 60-70.

Mulukutla, Bhanu Chandra, Salmaan Khan, Alex Lange, and Wei-Shou Hu. "Glucose metabolism in mammalian cell culture: new insights for tweaking vintage pathways." Trends in biotechnology 28, no. 9 (2010): 476-484.

Nadala, Elpidio Cesar, Yuanan Lu, and Philip C. Loh. "Primary culture of lymphoid, nerve, and ovary cells from Penaeus stylirostris and Penaeus vannamei." In Vitro Cellular & Developmental Biology-Animal 29, no. 8 (1993): 620-622.

Najafabadi, Ali K., R. D. Ellender, and B. L. Middlebrooks. "Analysis of shrimp hemolymph and ionic modification of a Penaeus cell culture formulation." Journal of aquatic animal health 4, no. 2 (1992): 143-148.

Nienow, Alvin W. "Re" Development of a Scale-Down Model of Hydrodynamic Stress to Study the Performance of an Industrial CHO Cell Line Under Simulated Production Scale Bioreactor Conditions"[Sieck, JB, Cordes, T., Budach, WE, Rhiel, MH, Suemeghy, Z., Leist, C., Villiger, TK, Morbidelli, M., Soos, M., 2013. Journal of Biotechnology 164, 41-49]." Journal of biotechnology 171 (2014): 82-84.

Osman, L. P., S. C. Mitchell, and R. H. Waring. "Cysteine, its metabolism and toxicity." Sulfur reports 20, no. 2 (1997): 155-172.

Owens, L. "Insight into the lymphoid organ of penaeid prawns: a review." Fish & shellfish immunology 29, no. 3 (2010): 367-377.

Ozturk, Sadettin S., and Bernhard O. Palsson. "Chemical decomposition of glutamine in cell culture media: effect of media type, pH, and serum concentration." Biotechnology progress 6, no. 2 (1990): 121-128.

Paine, Philip L., Terry W. Pearson, Louis JM Tluczek, and Samuel B. Horowitz. "Nuclear sodium and potassium." Nature 291, no. 5812 (1981): 258-261.

Permyakov, Eugene A., and Lawrence J. Berliner. "a-Lactalbumin: structure and function." FEBS letters 473, no. 3 (2000): 269-274.

Qiu, Mei, Yaling Wang, Xiaobo Wang, Lijun Sun, Riying Ye, Defeng Xu, Zhe Dai et al. "Effects of T-2 toxin on growth, immune function and hepatopancreas microstructure of shrimp (Litopenaeus vannamei)." Aquaculture 462 (2016): 35-39.

Rasmussen, Howard, David BP Goodman, Alan Tenenhouse, and A. B. Borle. "The Role of Cyclic AMP and Calcium in Cell Activatio." CRC critical reviews in biochemistry 1, no. 1 (1972): 95-148.

Rebhun, Lionel I. "Cyclic nucleotides, calcium, and cell division." International review of Cytology 49 (1977): 1-54.

Riley, Lisa G., Peter C. Wynn, Peter Williamson, and Paul A. Sheehy. "The role of native bovine α-lactalbumin in bovine mammary epithelial cell apoptosis and casein expression." Journal of dairy research 75, no. 3 (2008): 319-325.

Robertson, Lori, Bill Bray, Tzachi Samocha, and Addison Lawrence. "Reproduction of penaeid shrimp: an operations guide." CRC Hanbook of Mariculture, nd Edition 1 (1993): 107-132.

Rodriguez-Melendez, Rocio, and Janos Zempleni. "Regulation of gene expression by biotin." The Journal of nutritional biochemistry 14, no. 12 (2003): 680-690.

Rouiller, Yolande, Arnaud Périlleux, Marie-Noëlle Vesin, Matthieu Stettler, Martin Jordan, and Hervé Broly. "Modulation of mAb quality attributes using microliter scale fed-batch cultures." Biotechnology progress 30, no. 3 (2014): 571-583.

Roychoudhury, Shubhadeep, Peter Massanyi, Jozef Bulla, Manabendra Dutta Choudhury, Lubomir Straka, Norbert Lukac, Gregorz Formicki, Marianna Dankova, and Laszlo Bardos. "In vitro copper toxicity on rabbit spermatozoa motility, morphology and cell membrane integrity." Journal of Environmental Science and Health Part A 45, no. 12 (2010): 1482-1491.

Rubin, A. H., and Berbie Chu. "Reversible regulation by magnesium of chick embryo fibroblast proliferation." Journal of cellular physiology 94, no. 1 (1978): 13-19.

Salazar, Andrew, Michael Keusgen, and Jörg Von Hagen. "Amino acids in the cultivation of mammalian cells." Amino acids 48, no. 5 (2016): 1161-1171.

Sañudo-Wilhelmy, S. A., Gómez-Consarnau, L., Suffridge, C., & Webb, E. A. (2014). The role of B vitamins in marine biogeochemistry. Annual review of marine science, 6, 339-367.

Sato, Gordon H. "The role of serum in cell culture." Biochemical actions of hormones 3 (1975): 391-396.

Schanne, F. A., Agnes B. Kane, Ellora E. Young, and John L. Farber. "Calcium dependence of toxic cell death: a final common pathway." Science 206, no. 4419 (1979): 700-702.

Schneider, Markus, Ian W. Marison, and Urs Von Stockar. "The importance of ammonia in mammalian cell culture." Journal of biotechnology 46, no. 3 (1996): 161-185.

Schnellbaecher, A., Binder, D., Bellmaine, S., & Zimmer, A. (2019). Vitamins in cell culture media: Stability and stabilization strategies. Biotechnology and bioengineering, 116(6), 1537-1555.

Selvaraj, Venkatesan, and Jayaganesh Sankar. "Characterisation of magnesium toxicity, its influence on amino acid synthesis pathway and biochemical parameters of tea." Research Journal of Phytochemistry 4, no. 2 (2010): 67-77.

Shields, Jeffrey D., and Robert A. Boyd. "Atlas of lobster anatomy and histology." (2014).

Shike, Hiroko, C_ Shimizu, K. S. Klimpel, and J. C. Burns. "Expression of foreign genes in primary cultured cells of the blue shrimp Penaeus stylirostris." Marine Biology 137, no. 4 (2000): 605-611.

Somero, George N., and Paul H. Yancey. "Osmolytes and cell-volume regulation: Physiological and evolutionary principles." Comprehensive physiology (2010): 441-484.

Stankus, Austin. "State of world aquaculture 2020 and regional reviews: FAO webinar series." FAO Aquaculture Newsletter 63 (2021): 17-18.

Stanley, J. Steven, Jacob B. Griffin, and Janos Zempleni. "Biotinylation of histones in human cells: effects of cell proliferation." European Journal of Biochemistry 268, no. 20 (2001): 5424-5429.

Stepanyan, Ruben, Bettye Hollins, S. Erin Brock, and Timothy S. McClintock. "Primary culture of lobster (Homarus americanus) olfactory sensory neurons." Chemical senses 29, no. 3 (2004): 179-187.

Stipanuk, Martha H., John E. Dominy Jr, Jeong-In Lee, and Relicardo M. Coloso. "Mammalian cysteine metabolism: new insights into regulation of cysteine metabolism." The Journal of nutrition 136, no. 6 (2006): 1652S-1659S.

Suhaimi, Hazwani, Shuai Wang, and Diganta Bhusan Das. "Glucose diffusivity in cell culture medium." Chemical Engineering Journal 269 (2015): 323-327.

Tang, Hongping, Xintao Zhang, Weijian Zhang, Li Fan, Haibin Wang, Wen-Song Tan, and Liang Zhao. "Insight into the roles of tyrosine on rCHO cell performance in fed-batch cultures." Applied microbiology and biotechnology 103, no. 16 (2019): 6483-6494.

Tapay, Lourdes M., Yuanan Lu, James A. Brock, Elpidio Cesar B. Nadala Jr, and Philip C. Loh. "Transformation of primary cultures of shrimp (Penaeus stylirostris) lymphoid (Oka) organ with simian virus-40 (T) antigen." Proceedings of the Society for Experimental Biology and Medicine 209, no. 1 (1995): 73-78.

Taylor, Milton W. "A history of cell culture." In Viruses and man: a history of interactions, pp. 41-52. Springer, Cham, 2014.

Timasheff, Serge N. "The control of protein stability and association by weak interactions with water: how do solvents affect these processes?." Annual review of biophysics and biomolecular structure 22, no. 1 (1993): 67-97.

Todaro, George J., Gerald K. Lazar, and Howard Green. "The initiation of cell division in a contactinhibited mammalian cell line." Journal of Cellular and Comparative Physiology 66, no. 3 (1965): 325-333.

Tong, Shang-liang, and Hong-Zhi Miao. "Attempts to initiate cell cultures from Penaeus chinensis tissues." Aquaculture 147, no. 3-4 (1996): 151-157.

Toullec, J. Y., Y. Crozat, J. Patrois, and P. Porcheron. "Development of primary cell cultures from the penaeid shrimps Penaeus vannamei and P. indicus." Journal of Crustacean Biology 16, no. 4 (1996): 643-649.

Toullec, Jean-Yves. "Crustacean primary cell culture: a technical approach." Methods in cell science 21, no. 4 (1999): 193-198.

Tritsch, G. L., and G. E. Moore. "Spontaneous decomposition of glutamine in cell culture media." Experimental cell research 28, no. 2 (1962): 360-364.

Van de Braak, C. B. T., M. H. A. Botterblom, N. V. Taverne, W. B. Van Muiswinkel, J. H. W. M. Rombout, and W. P. W. Van der Knaap. "The roles of haemocytes and the lymphoid organ in the clearance of injected Vibrio bacteria in Penaeus monodon shrimp." Fish & shellfish immunology 13, no. 4 (2002): 293-309.

Van Why, Scott K., and Norman J. Siegel. "Heat shock proteins in renal injury and recovery." Current opinion in nephrology and hypertension 7, no. 4 (1998): 407-412.

Villena, Alberto J. "Applications and needs of fish and shellfish cell culture for disease control in aquaculture." Reviews in fish biology and Fisheries 13, no. 1 (2003): 111-140.

Walker, A. "Crustacean aquaculture." Proceedings of the Nutrition Society 34, no. 1 (1975): 65-73.

Wang, Chung-Hsiung, Hsi-Nan Yang, Chih-Yuan Tang, Chien-Hsin Lu, Guang-Hsiung Kou, and Chu-Fang Lo. "Ultrastructure of white spot syndrome virus development in primary lymphoid organ cell cultures." Diseases of Aquatic Organisms 41, no. 2 (2000): 91-104.

Waymouth, Charity. "Osmolality of mammalian blood and of media for culture of mammalian cells." In vitro 6, no. 2 (1970): 109-127.

Weil, Brent R., Aaron M. Abarbanell, Jeremy L. Herrmann, Yue Wang, and Daniel R. Meldrum. "High glucose concentration in cell culture medium does not acutely affect human mesenchymal stem cell growth factor production or proliferation." American Journal of Physiology-Regulatory, Integrative and Comparative Physiology 296, no. 6 (2009): R1735-R1743.

Weltin, Andreas, Kinga Slotwinski, Jochen Kieninger, Isabella Moser, Gerhard Jobst, Marcus Wego, Ralf Ehret, and Gerald A. Urban. "Cell culture monitoring for drug screening and cancer research: a transparent, microfluidic, multi-sensor microsystem." Lab on a Chip 14, no. 1 (2014): 138-146.

Wessman, S. J., and R. L. Levings. "Benefits and risks due to animal serum used in cell culture production." Developments in biological standardization 99 (1999): 3-8.

Wu, Guoyao. "Functional amino acids in growth, reproduction, and health." Advances in nutrition 1, no. 1 (2010): 31-37.

Yamane, Isao, Osamu Murakami, and Miwa Kato. "Role of bovine albumin in a serum-free suspension cell culture medium." Proceedings of the Society for Experimental Biology and Medicine 149, no. 2 (1975): 439-442.

Yao, Tatsuma, and Yuta Asayama. "Animal-cell culture media: History, characteristics, and current issues." Reproductive medicine and biology 16, no. 2 (2017): 99-117.

Yaqoob, Parveen, and Philip C. Calder. "Glutamine requirement of proliferating T lymphocytes." Nutrition 13, no. 7-8 (1997): 646-651.

Yki-Järvinen, Hannele. "Glucose toxicity." Endocrine reviews 13, no. 3 (1992): 415-431.

Yu, Marcella, Zhilan Hu, Efren Pacis, Natarajan Vijayasankaran, Amy Shen, and Feng Li. "Understanding the intracellular effect of enhanced nutrient feeding toward high titer antibody production process." Biotechnology and bioengineering 108, no. 5 (2011): 1078-1088.

Zaken, Varda, Ron Kohen, and Asher Ornoy. "Vitamins C and E improve rat embryonic antioxidant defense mechanism in diabetic culture medium." Teratology 64, no. 1 (2001): 33-44.

Zielke, H. Ronald, Carol L. Zielke, and Pinar T. Ozand. "Glutamine: a major energy source for cultured mammalian cells." In Federation proceedings, vol. 43, no. 1, pp. 121-125. 1984.

## Claims

1. A cell culture medium comprising: 1) an osmolality of 835 +/- 55 mmol/kg, 2) a pH ranging between 7.2 and 7.4, 3) the amino acids: glycine, L-alanine, L-aspartic acid, L-asparagine, L-glutamic acid, L-alanyl-L-glutamine, L-proline and L-serine, 4) the vitamins: choline chloride, calcium pantothenate, folic acid, nicotinamide, pyridoxal hydrochloride, riboflavin, thiamine hydrochloride and inositol, and 5) the supplements: glucose, lactalbumin, and ultrafiltered crustacean hemolymph.

2. A cell culture medium according to claim 1 which further comprises the amino acids L-cysteine and L-tyrosine, and the vitamin biotin.

3. A cell culture medium according to claims 1-2 wherein said osmolality is determined by the addition of the salts: sodium chloride, magnesium sulphate, magnesium chloride.6H₂O, calcium chloride.2H₂0 and potassium chloride.

4. A cell culture medium according to claims 1-3 wherein said pH is determined by the addition of sodium bicarbonate and the presence of CO₂ in the air.

5. A cell culture medium according to claims 3-4 wherein said salts have the following concentrations: 8.27 +/- 0.8 g/L sodium chloride, 0.065 g/L magnesium sulphate, 0.064 g/L magnesium chloride.6H₂O, 0.27 g/L calcium chloride.2H₂0 and 0.16 g/L potassium chloride.

6. A cell culture medium according to claims 4-5 wherein said pH is determined by the addition of the following concentrations of sodium bicarbonate and CO₂: 2 g/L sodium bicarbonate and the presence of 5% CO₂ in the air.

7. A cell culture medium according to claims 1-6 comprising the following concentrations of amino acids, vitamins, and supplements: 3.75-37.5 mg/L glycine, 4.45-44.5 mg/L L-alanine, 6.65-66.5 mg/L L-aspartic acid, 6.6-66 mg/L L-asparagine,7.35-73.5 mg/L L-glutamic acid, 217.22-651.66 mg/L L-alanyl-L-glutamine, 5.75-57.5 mg/L L-proline, 5.25-52.5 mg/L L-serine, 10.0-50.0 mg/L L-cysteine, 10.0-50.0 mg/L L-tyrosine, 1.0-2.0 mg/L choline chloride, 1.0-2.0 mg/L calcium pantothenate, 1.0-2.0 mg/L folic acid, 1.0-2.0 mg/L nicotinamide, 1.0-2.0 mg/L pyridoxal hydrochloride, 0.1-0.2 mg/L riboflavin, 1.0-2.0 mg/L thiamine hydrochloride and 2-4 mg/L inositol, 0.02-0.2 mg/L biotin, 1-2 g/L glucose, 1-2 g/L lactalbumin, and 5% of final volume ultrafiltered crustacean hemolymph.

8. A cell culture medium according to claim 7 comprising the following concentrations of amino acids, vitamins, and supplements:: 11.25 mg/L glycine, 13.35 mg/L L-alanine, 19.95 mg/L L-aspartic acid, 19.80 mg/L L-asparagine,22.05 mg/L L-glutamic acid, 434.44 mg/L L-alanyl-L-glutamine, 17.25 mg/L L-proline, 15.75 mg/L L-serine, 30.0 mg/L L-cysteine, 30.0 mg/L L-tyrosine, 2 mg/L choline chloride, 2 mg/L calcium pantothenate, 2 mg/L folic acid, 2 mg/L nicotinamide, 2 mg/L pyridoxal hydrochloride, 0.2 mg/L riboflavin, 2 mg/L thiamine hydrochloride and 4.0 mg/L inositol, 0.11 mg/L biotin, 1.5 g/L glucose, 1.5 g/L lactalbumin, and 5% of final volume ultrafiltered crustacean hemolymph;

9. A cell culture medium according to claims 1-8 which further comprises at least one of the following compounds: penicillin, streptomycin, gentamicin, amphotericin B.

10. A cell culture medium according to claims 1-8 which further comprises the following concentrations of compounds: 100000 U/L penicillin, 100 mg/L streptomycin, 50 mg/L gentamicin and 0.25 mg/L amphotericin B.

11. Use of a cell culture medium according to claims 1-10 to maintain the viability of cells and/or to proliferate cells taken from a crustacean in vitro.

12. Use according to claim 11 wherein said cells taken from a crustacean are cells or explants taken from a lymphoid organ, an embryo, an ovary, or a hematopoietic tissue of said crustacean.

13. Use according to claims 11-12 wherein said crustacean is a shrimp or a lobster.

14. A method to obtain a subculture from the proliferating cells obtained via the usage of a cell culture medium according to claim 11 comprising: 1) washing said proliferating cells using a buffered washing solution to remove bivalent ions that are crucial for cell attachment, and 2) detaching said proliferating cells from a plate coated with coating material with an enzyme capable of cleaving said coating material, wherein said coating material is collagen IV, wherein said enzyme is collagenase IV and wherein said buffered washing solution comprises: a pH ranging between 7.2 and 7.4, an osmolality of 835±55 mmol/kg, 371.9±25 mM NaCl, 8 mM Na₂HPO₄, 2 mM KH₂PO₄, and 2.7 mM KCl.

## Patentansprüche

1. Zellkulturmedium, umfassend: 1) eine Osmolalität von 835 +/- 55 mmol/kg, 2) einen pH-Wert zwischen 7,2 und 7,4, 3) die Aminosäuren Glycin, L-Alanin, L-Asparaginsäure, L-Asparagin, L-Glutaminsäure, L-Alanyl-L-glutamin, L-Prolin und L-Serin, 4) die Vitamine Cholinchlorid, Calciumpantothenat, Folsäure, Nicotinamid, Pyridoxalhydrochlorid, Riboflavin, Thiaminhydrochlorid und Inositol und 5) die Zusatzstoffe Glucose, Lactalbumin und ultrafiltrierte Krustentier-Hämolymphe.

2. Zellkulturmedium nach Anspruch 1, das ferner die Aminosäuren L-Cystein und L-Tyrosin sowie das Vitamin Biotin umfasst.

3. Zellkulturmedium nach Anspruch 1 und 2, wobei die Osmolalität durch die Zugabe der Salze Natriumchlorid, Magnesiumsulfat, Magnesiumchlorid.6H₂O, Calciumchlorid.2H₂O und Kaliumchlorid bestimmt wird.

4. Zellkulturmedium nach Anspruch 1 bis 3, wobei der pH durch die Zugabe von Natriumhydrogencarbonat und das Vorliegen von CO₂ in der Luft bestimmt wird.

5. Zellkulturmedium nach Anspruch 3 und 4, wobei die Salze die folgenden Konzentrationen aufweisen: 8,27 +/-0,8 g/l Natriumchlorid, 0,065 g/l Magnesiumsulfat, 0,064 g/l Magnesiumchlorid.6H₂O, 0,27 g/l Calciumchlorid.2H₂O und 0,16 g/l Kaliumchlorid.

6. Zellkulturmedium nach Anspruch 4 und 5, wobei der pH durch die Zugabe der folgenden Konzentrationen an Natriumhydrogencarbonat und CO₂ bestimmt wird: 2 g/l Natriumhydrogencarbonat und das Vorliegen von 5 % CO₂ in der Luft.

7. Zellkulturmedium nach Anspruch 1 bis 6, umfassend die folgenden Konzentrationen an Aminosäuren, Vitaminen und Zusatzstoffen: 3,75-37,5 mg/l Glycin, 4,45-44,5 mg/l L-Alanin, 6,65-66,5 mg/L L-Asparaginsäure, 6,6-66 mg/l L-Asparagin, 7,35-73,5 mg/l L-Glutaminsäure, 217,22-651,66 mg/l L-Alanyl-L-glutamin, 5,75-57,5 mg/l L-Prolin, 5,25-52,5 mg/l L-Serin, 10,0-50,0 mg/l L-Cystein, 10,0-50,0 mg/l L-Tyrosin, 1,0-2,0 mg/l Cholinchlorid, 1,0-2,0 mg/l Calciumpantothenat, 1,0-2,0 mg/l Folsäure, 1,0-2,0 mg/l Nicotinamid, 1,0-2,0 mg/l Pyridoxalhydrochlorid, 0,1-0,2 mg/l Riboflavin, 1,0-2,0 mg/l Thiaminhydrochlorid und 2-4 mg/l Inositol, 0,02-0,2 mg/l Biotin, 1-2 g/l Glucose, 1-2 g/l Lactalbumin und 5 % Endvolumen ultrafiltrierte Krustentier-Hämolymphe.

8. Zellkulturmedium nach Anspruch 7, umfassend die folgenden Konzentrationen an Aminosäuren, Vitaminen und Zusatzstoffen: 11,25 mg/l Glycin, 13,35 mg/l L-Alanin, 19,95 mg/l L-Asparaginsäure, 19,80 mg/l L-Asparagin, 22,05 mg/l L-Glutaminsäure, 434,44 mg/l L-Alanyl-L-glutamin, 17,25 mg/l L-Prolin, 15,75 mg/l L-Serin, 30,0 mg/l L-Cystein, 30,0 mg/l L-Tyrosin, 2 mg/l Cholinchlorid, 2 mg/l Calciumpantothenat, 2 mg/l Folsäure, 2 mg/l Nicotinamid, 2 mg/l Pyridoxalhydrochlorid, 0,2 mg/l Riboflavin, 2 mg/l Thiaminhydrochlorid und 4,0 mg/l Inositol, 0,11 mg/l Biotin, 1,5 g/l Glucose, 1,5 g/l Lactalbumin und 5 % Endvolumen ultrafiltrierte Krustentier-Hämolymphe.

9. Zellkulturmedium nach Anspruch 1 bis 8, das ferner mindestens eine der folgenden Verbindungen umfasst: Penicillin, Streptomycin, Gentamicin, Amphotericin B.

10. Zellkulturmedium nach Anspruch 1 bis 8, das ferner die folgenden Verbindungskonzentrationen umfasst: 100000 U/l Penicillin, 100 mg/l Streptomycin, 50 mg/l Gentamicin und 0,25 mg/l Amphotericin B.

11. Verwendung eines Zellkulturmediums nach Anspruch 1 bis 10 zur Aufrechterhaltung der Lebensfähigkeit von Zellen und/oder zur In-vitro-Proliferation von Zellen, die einem Krustentier entnommen sind.

12. Verwendung nach Anspruch 11, wobei es sich bei den einem Krustentier entnommenen Zellen um Zellen oder Explantate handelt, die einem lymphatischen Organ, einem Embryo, einem Ovar oder einem hämatopoetischen Gewebe des Krustentiers entnommen wurden.

13. Verwendung nach Anspruch 11 und 12, wobei es sich bei dem Krustentier um eine Garnele oder einen Hummer handelt.

14. Verfahren zur Gewinnung einer Subkultur aus den proliferierenden Zellen, die über die Anwendung eines Zellkulturmediums nach Anspruch 11 erhalten wurden, umfassend: 1) Waschen der proliferierenden Zellen unter Verwendung einer gepufferten Waschlösung zur Entfernung zweiwertiger Ionen zu entfernen, die für die Zellanbindung entscheidend sind, und 2) Ablösen der proliferierenden Zellen von einer mit Beschichtungsmaterial beschichteten Platte, mit einem Enzym, das in der Lage ist, das Beschichtungsmaterial zu spalten, wobei es sich bei dem Beschichtungsmaterial um Kollagen IV handelt, wobei es sich bei dem Enzym um Kollagenase IV handelt und wobei die gepufferte Waschlösung Folgendes umfasst: ein pH-Wert zwischen 7,2 und 7,4, eine Osmolalität von 835±55 mmol/kg, 371,9±25 mM NaCl, 8 mM Na₂HPO₄, 2 mM KH₂PO₄ und 2,7 mM KCl.

## Revendications

1. Milieu de culture cellulaire comprenant : 1) une osmolalité de 835 +/- 55 mmol/kg, 2) un pH compris entre 7,2 et 7,4, 3) les acides aminés : glycine, L-alanine, acide L-aspartique, L-asparagine, acide L-glutamique, L-alanyl-L-glutamine, L-proline et L-sérine, 4) les vitamines : chlorure de choline, pantothénate de calcium, acide folique, nicotinamide, chlorhydrate de pyridoxal, riboflavine, chlorhydrate de thiamine et inositol, et 5) les suppléments : glucose, lactalbumine et hémolymphe de crustacés ultrafiltrée.

2. Milieu de culture cellulaire selon la revendication 1, qui comprend en outre les acides aminés L-cystéine et L-tyrosine, et la vitamine biotine.

3. Milieu de culture cellulaire selon les revendications 1-2 dans lequel ladite osmolalité est déterminée par ajout des sels : chlorure de sodium, sulfate de magnésium, chlorure de magnésium 6H₂O, chlorure de calcium 2H₂O et chlorure de potassium.

4. Milieu de culture cellulaire selon la revendication 1-3, dans lequel ledit pH est déterminé par l'ajout de bicarbonate de sodium et la présence de CO₂ dans l'air.

5. Milieu de culture cellulaire selon les revendications 3-4, dans lequel lesdits sels possèdent les concentrations suivantes : 8,27 +/- 0,8 g/L de chlorure de sodium, 0,065 g/L de sulfate de magnésium, 0,064 g/L de chlorure de magnésium 6H₂O, 0,27 g/L de chlorure de calcium 2H₂0 et 0,16 g/L de chlorure de potassium.

6. Milieu de culture cellulaire selon les revendications 4-5, dans lequel ledit pH est déterminé par l'ajout des concentrations suivantes de bicarbonate de sodium et de CO₂: 2 g/L de bicarbonate de sodium et présence de 5 % de CO₂ dans l'air.

7. Milieu de culture cellulaire selon la revendication 1-6, comprenant les concentrations suivantes d'acides aminés, de vitamines, et de suppléments : 3,75-37,5 mg/L de glycine, 4,45-44,5 mg/L de L-alanine, 6,65-66,5 mg/L d'acide L-aspartique, 6,6-66 mg/L de L-asparagine, 7,35-73,5 mg/L d'acide L-glutamique, 217,22-651,66 mg/L de L-alanyl-L-glutamine, 5,75-57,5 mg/L de L-proline, 5,25-52,5 mg/L de L-sérine, 10,0-50,0 mg/L de L-cystéine, 10,0-50,0 mg/L de L-tyrosine, 1,0-2,0 mg/L de chlorure de choline, 1,0-2,0 mg/L de pantothénate de calcium, 1,0-2,0 mg/L d'acide folique, 1,0-2,0 mg/L de nicotinamide, 1,0-2,0 mg/L de chlorhydrate de pyridoxal, 0,1-0,2 mg/L de riboflavine, 1,0-2,0 mg/L de chlorhydrate de thiamine et 2-4 mg/L d'inositol, 0,02-0,2 mg/L de biotine, 1-2 g/L de glucose, 1-2 g/L de lactalbumine et 5 % du volume final d'hémolymphe de crustacés ultrafiltrée.

8. Milieu de culture cellulaire selon la revendication 7, comprenant les concentrations suivantes d'acides aminés, de vitamines, et de suppléments : 11,25 mg/L de glycine, 13,35 mg/L de L-alanine, 19,95 mg/L d'acide L-aspartique, 19,80 mg/L de L-asparagine, 22,05 mg/L d'acide L-glutamique, 434,44 mg/L de L-alanyl-L-glutamine, 17,25 mg/L de L-proline, 15,75 mg/L de L-sérine, 30,0 mg/L de L-cystéine, 30,0 mg/L de L-tyrosine, 2 mg/L de chlorure de choline, 2 mg/L de pantothénate de calcium, 2 mg/L d'acide folique, 2 mg/L de nicotinamide, 2 mg/L de chlorhydrate de pyridoxal, 0,2 mg/L de riboflavine, 2 mg/L de chlorhydrate de thiamine et 4,0 mg/L d'inositol. 0,11 mg/L de biotine, 1,5 g/L de glucose, 1,5 g/L de lactalbumine et 5 % du volume final d'hémolymphe de crustacés ultrafiltrée.

9. Milieu de culture cellulaire selon les revendications 1-8 qui comprend en outre au moins l'un des composés suivants : pénicilline, streptomycine, gentamicine, amphotéricine B.

10. Milieu de culture cellulaire selon la revendication 1-8, qui comprend en outre les concentrations de composés suivantes : 100 000 U/L de pénicilline, 100 mg/L de streptomycine, 50 mg/L de gentamicine et 0,25 mg/L d'amphotéricine B.

11. Utilisation d'un milieu de culture cellulaire selon les revendications 1-10 pour maintenir la viabilité de cellules et/ou pour faire proliférer des cellules prélevées d'un crustacé *in vitro.*

12. Utilisation selon la revendication 11, dans laquelle lesdites cellules prélevées d'un crustacé sont des cellules ou des explants prélevés d'un organe lymphoïde, un embryon, un ovaire ou un tissu hématopoïétique dudit crustacé.

13. Utilisation selon les revendications 11-12 dans laquelle ledit crustacé est une crevette ou un homard.

14. Procédé pour obtenir une sous-culture à partir des cellules en prolifération obtenues par l'utilisation d'un milieu de culture cellulaire selon la revendication 11 comprenant : 1) lavage desdites cellules en prolifération à l'aide d'une solution de lavage tamponnée pour éliminer des ions bivalents qui sont cruciaux pour la fixation des cellules, et 2) détachement desdites cellules en prolifération d'une plaque revêtue par un matériau de revêtement avec une enzyme capable de cliver ledit matériau de revêtement, ledit matériau de revêtement étant le collagène IV, ladite enzyme étant la collagénase IV et où ladite solution de lavage tamponnée comprend : un pH compris entre 7,2 et 7,4, une osmolalité de 835 ± 55 mmol/kg, 371,9 ± NaCl 25 mM, Na₂HPO₄ 8 mM, KH₂PO₄ 2 mM et KCl 2,7 mM.
